# FASCICULE DE BREVET EUROPEEN

(11) **EP 1 453 598 B1**
(45) Date de publication et mention de la délivrance du brevet: **11.06.2008**
(21) Numéro de dépôt: 02804595.3
(22) Date de dépôt: 02.12.2002
(51) Int. Cl.: B01F 17/00, A01N 25/04, A01N 25/30, C11D 3/37, C10M 173/00, D21H 17/44

(54) **PROCEDE DE PREPARATION D UNE EMULSION MULTIPLE DE TYPE EAU/HUILE/EAU**
VERFAHREN ZUR HERSTELLUNG EINER MEHRFACH-WASSER/ÖL/WASSER-EMULSION
METHOD FOR PREPARING A MULTIPLE WATER/OIL/WATER EMULSION

(30) Priorité: 11.12.2001 FR 0115982
(43) Date de publication de la demande: 08.09.2004
(73) Titulaire: RHODIA CHIMIE, 92512 Boulogne Billancourt Cedex (FR)
(72) Inventeur: MERCIER, Jean-Michel, F-60270 GOUVIEUX (FR); VALLIER, Emmanuelle, F-95150 TAVERNY (FR)
(74) Mandataire: Boittiaux, Vincent
(86) Numéro de dépôt international: PCT/FR2002/004127
(87) Numéro de publication internationale: WO 2003/049846

(56) Documents cités:
- WO-A-01/14505
- US-A- 4 931 210
- LUCA DE M ET AL: "A STABLE W/O/W MULTIPLE EMULSION" COSMETICS & TOILETRIES, WHEATON, IL, US, vol. 105, novembre 1990 (1990-11), pages 65-66,69, XP001095853 ISSN: 0361-4387

## Description

La présente invention a pour objet un procédé de préparation d'une émulsion multiple de type eau / huile / eau dans lequel on ne met avantageusement en oeuvre qu'un seul réacteur.

Les procédés classiques de préparation d'émulsions multiples eau / huile / eau consistent tout d'abord à préparer l'émulsion inverse eau dans huile dans un premier réacteur. Puis, dans un second réacteur, est préparée une phase aqueuse externe. On ajoute ensuite l'émulsion inverse obtenue précédemment dans la phase aqueuse externe. Ce type de processus ne pose pas de problème majeur, notamment lorsque l'émulsion inverse est peu visqueuse, ce qui se traduit par le fait que l'huile constituant la phase organique de l'émulsion inverse est fluide, ou bien encore lorsque la concentration de phase aqueuse dispersée dans la phase organique de l'émulsion multiple reste relativement peu élevée (inférieure à 50 % en poids de l'ensemble), voire la combinaison des deux. Il présente toutefois un inconvénient dû au fait que deux réacteurs sont nécessaires pour faire cette opération, dont l'un d'eux nécessitera la mise en place de moyens particuliers pour en assurer le nettoyage correct entre deux campagnes de production.

Par contre, lorsque la différence entre la viscosité de l'émulsion inverse et celle de la phase aqueuse externe à laquelle elle est ajoutée, est importante, la mise en oeuvre du procédé décrit ci-dessus devient plus complexe pour un résultat moins satisfaisant.

Tout d'abord, l'un des problèmes réside dans le caractère difficilement manipulable des émulsions inverses de viscosité importante.

Par ailleurs, on rencontre des difficultés pour ce qui concerne l'agitation. En effet, dans les conditions où la différence de viscosité entre les phases aqueuse et organique est élevée, on observe une baisse d'efficacité de l'agitation, en d'autres termes, le cisaillement produit par l'agitation et qui est transmis par la phase continue de l'émulsion, est beaucoup moins efficace. Par conséquent, afin d'obtenir une taille de gouttelettes satisfaisante, il est nécessaire de mettre en oeuvre des moyens d'agitation développant considérablement plus d'énergie que les moyens classiques. Mais l'emploi de ce type d'agitation n'est pas sans conséquence sur l'émulsion inverse, puisque, dans de telles conditions d'agitation, les gouttelettes de l'émulsion inverse peuvent, elles-mêmes, être cisaillées entraînant un risque non négligeable de libération de la matière active encapsulée dans la phase aqueuse interne de l'émulsion multiple.

Une solution à ce problème de mise en oeuvre a consisté à utiliser des polymères thermoépaississants. Ces polymères, introduits dans la phase aqueuse externe, développent une viscosité importante lorsque la température du milieu aqueux dans lequel ils sont, est supérieure à leur température de seuil. Plus particulièrement, ces polymères sont solubles dans l'eau à température ambiante, et au-delà de la température-seuil, une partie du polymère devient hydrophobe (partie thermosensible) : le polymère forme ainsi un réseau physique à l'échelle microscopique, ce qui se traduit à l'échelle macroscopique par une augmentation de la viscosité. Par conséquent, la différence de viscosité entre la phase aqueuse externe et l'émulsion inverse est limitée, et l'on observe alors une augmentation de l'efficacité du cisaillement par un meilleur transfert de la phase continue de l'émulsion à la phase discontinue. En conséquence les moyens d'agitation susceptibles d'être mis en oeuvre peuvent être moins énergétiques et le problème de la libération de la matière active emprisonnée dans la phase aqueuse interne est aussi limité.

Un premier objet de la présente invention consiste à apporter une alternative à l'emploi de polymères thermoépaisssants.

Par ailleurs, un autre objectif de la présente invention est de proposer un procédé de préparation d'émulsions multiples eau / huile / eau qui puisse être mis en oeuvre avec des émulsions multiples présentant des gammes de viscosité étendues.

Un autre objet de l'invention est de pouvoir disposer d'un procédé évitant de manière efficace toute libération de la matière active encapsulée dans la phase aqueuse interne, durant la préparation de l'émulsion.

Ces buts et d'autres sont atteints par la présente invention qui a donc pour objet un procédé de préparation d'une émulsion multiple comprenant une émulsion inverse constituée d'une phase aqueuse interne dispersée dans une phase organique interne; l'émulsion inverse étant dispersée dans une phase aqueuse externe ;
□ la phase aqueuse interne comprenant éventuellement au moins une matière active hydrophile ; la phase organique interne comprenant éventuellement au moins une matière active hydrophobe ;
□ l'émulsion inverse comprenant au moins un tensioactif et/ou au moins un polymère amphiphile interne(s) ;
□ la phase aqueuse externe comprenant au moins un tensioactif et/ou au moins un polymère amphiphile externe(s) et éventuellement au moins une matière active hydrophile ;
le procédé selon l'invention consistant à mettre en oeuvre les étapes suivantes :
a) on prépare l'émulsion inverse ;
b) on prépare la phase aqueuse externe ;
c) on introduit la phase aqueuse externe dans l'émulsion inverse, sans agitation ;
d) on agite l'ensemble ;
e) on dilue éventuellement l'émulsion multiple concentrée ainsi obtenue, par ajout, sous agitation, d'une phase aqueuse de dilution comprenant au moins un tensioactif et/ou au moins un polymère amphiphile externe(s), afin d'obtenir une émulsion multiple diluée.

Le procédé selon l'invention présente l'avantage de permettre l'accès à des émulsions multiples dont la taille moyenne des gouttelettes peut être faible alors que les conditions opératoires sont a priori défavorables, comme par exemple l'utilisation d'une émulsion inverse concentrée et/ou dont la phase organique est visqueuse.

Par ailleurs, le procédé selon l'invention ne nécessite plus la mise en oeuvre de deux réacteurs en cascade, comme c'est le cas pour les procédés classiques. Par conséquent, le procédé selon l'invention s'affranchit des problèmes liés à la manipulation (transfert) de l'émulsion inverse dans un autre réacteur. De plus, les problèmes de nettoyage du réacteur utilisé pour la préparation de l'émulsion inverse sont résolus dans le cas de la présente invention car l'émulsion multiple finale obtenue est une émulsion dont la phase externe est aqueuse.

En outre, le procédé selon l'invention permet d'obtenir des émulsions multiples de diverses concentrations. En effet, non seulement l'émulsion multiple obtenue à l'issue du procédé selon l'invention se trouve sous une forme concentrée mais elle peut encore faire l'objet d'une dilution ultérieure, et cela sans aucune difficulté, ni perte des caractéristiques de taille des gouttelettes de l'émulsion multiple avant dilution.

Mais d'autres avantages de la présente invention apparaîtront plus clairement à la lecture de la description et des exemples qui vont suivre.

Il est précisé que dans ce qui va suivre, le terme polymères est employé à la fois pour désigner des homopolymères ou des copolymères.

De plus, pour différencier les phases de l'émulsion multiple et leurs divers constituants, les uns des autres, on appellera tout d'abord phase aqueuse interne, phase organique interne et tensioactif et/ou polymère amphiphile internes, les phases, tensioactifs et polymères présents dans l'émulsion inverse. Par ailleurs, on appellera phase aqueuse externe et tensioactif et/ou polymère amphiphile externes, les phases, tensioactifs et polymères présents dans la phase aqueuse externe de l'émulsion multiple. Il est précisé que si la phase aqueuse externe de l'émulsion multiple comprend une phase organique dispersée, différente d'une émulsion inverse, celle-ci sera qualifiée de phase organique externe.

Pour des raisons de clarté de l'exposé, les éléments constitutifs de l'émulsion multiple vont être détaillés, et tout d'abord les éléments constituants l'émulsion inverse.

Ainsi, comme cela a été indiqué auparavant, la phase aqueuse interne comprend éventuellement au moins une matière active hydrophile.

Cette matière active hydrophile peut se présenter sous une forme liquide ; sous une forme solubilisée dans un solvant miscible à l'eau comme l'éthanol, le propylène glycol, le glycérol ; ou encore sous une forme solide.

La teneur en matière active hydrophile est plus particulièrement comprise entre 0,1 et 50 % en poids de la phase aqueuse interne, et de préférence comprise entre 0,1 et 20 % en poids de la phase aqueuse interne.

Etant donné le nombre important de domaines dans lesquels les émulsions multiples selon l'invention sont susceptibles d'être utilisées, de nombreuses matières actives peuvent convenir à la mise en oeuvre de l'invention.

A titre d'exemples de matières actives utilisables dans le domaine de la cosmétique, on peut citer les substances qui ont un effet cosmétique, un effet thérapeutique ou toute autre substance utilisable pour le traitement de la peau et/ou du cheveu.

Ainsi, on peut utiliser, en tant que matière active des agents conditionneurs de la peau et/ou du cheveu, comme notamment les polymères comprenant des ammonium quaternaires qui peuvent éventuellement être engagés dans des hétérocycles (composés du type des quaternium, polyquaternium, etc.) ; des agents humectants ; des agents fixants (styling) qui sont plus particulièrement choisis parmi des polymères (homo-, co- ou ter-polymères par exemple acrylamide, acrylamide/acrylate de sodium, polystyrène sulfonate, etc.), les polymères cationiques, la polyvinylpyrrolidone, l'acétate de polyvinyle,etc.

Il est de même possible d'utiliser des agents colorants ; des agents astringents, utilisables dans les déodorants et qui sont plus particulièrement des sels d'aluminium, de zirconium ; des agents antibactériens ; des agents anti-inflammatoires, des agents anesthésiants, des filtres solaires, etc.

On peut aussi citer les α- et β- hydroxyacides, comme les acides citrique, lactique, glycolique, salicylique ; les acides dicarboxyliques, de préférence insaturés, et comprenant 9 à 16 atomes, de carbone comme l'acide azélaique ; la vitamine C et ses dérivés, notamment les dérivés glycosylés et phosphatés ; les biocides notamment cationiques (Glokill PQ, Rhodoquat RP50, commercialisés par Rhodia Chimie), comme matières actives convenables dans les formulations cosmétiques.

Dans le domaine de l'alimentaire, on peut citer par exemple les sels divalents de calcium (les phosphates, les chlorures, etc.) utilisés comme agent réticulant de polymères texturant comme les alginates, les carraghénannes ; le bicarbonate de sodium, entre autres.

Dans le domaine des matières actives phytosanitaires, on peut utiliser des pesticides hydrophiles ou des éléments nutritifs hydrophiles favorisant la croissance et le développement des plantes.

Parmi les matières actives convenables, on peut notamment citer les matières actives herbicides suivantes, sous la forme ou non de sels organiques ou inorganiques : les dérivés aminophosphates ou aminophosphonates, Acifluorfen, Asulam, Benazolin, Bentazon, Bialaphos, Bispyribac, Bromacil, Bromoxynil, Chloramben, Clopyralid, 2,4-D, 2,4-Db, Dalapon, Dicamba, Dichlorprop, Diclofop, Difenzoquat, Diquat, Endothall, Fenac, Fenoxaprop, Flamprop, Fluazifop, Fluoroglycofen, Fluroxypyr, Fomesafen, Fosamine, Haloxyfop, loxynil, Mcpa, Mcpb, Mecoprop, Methylarsonic Acid, Naptalam, Nonanoic Acid, Paraquat, Picloram, Sulfamic Acid, ou leurs mélanges.

De préférence, la matière active est choisie parmi les dérivés aminophosphates ou aminophosphonates, sous la forme de sels organiques ou inorganiques, comme le glyphosate, le sulphosate, le glufosinate.

Pour ce qui a trait au domaine de l'exploitation ou la construction de puits de gisement de pétrole ou de gaz, la présente invention peut être mise en oeuvre pour des matières actives hydrophiles utilisables notamment lors des opérations cimentation, complétion, forage et stimulation des puits (par exemple la fracturation). A titre d'exemples de matières actives utilisables dans ce domaine, on peut mentionner des catalyseurs de réticulation de compositions cimentaires, comme par exemple les sels de lithium, tels que le chlorure, l'acétate. On peut de même citer des composés susceptibles, entre autres, de dégrader les polysaccharides, comme par exemple les acides carboxyliques (notamment l'acide citrique), des enzymes (notamment les cellulases), des oxydants.

Dans le domaine des silicones, on peut citer par exemple les sels de calcium, la potasse, utilisés habituellement comme agents réticulants.

A titre de matières actives convenables dans le domaine de la fabrication du papier, on peut citer notamment le chlorure de calcium, l'acide chlorhydrique.

Conformément à un mode de réalisation particulièrement avantageux, la phase aqueuse interne peut comprendre au moins un additif choisi parmi les sels tels que les halogénures de métaux alcalins ou alcalino-terreux (comme le chlorure de sodium, le chlorure de calcium), ou les sulfates de métaux alcalin ou alcalino-terreux (comme le sulfate de sodium), ou leurs mélanges. A titre d'additif possible de la phase aqueuse interne, on peut aussi citer les sucres, comme le glucose par exemple, ou encore les polysaccharides, comme notamment le dextran, ou leurs mélanges.

La concentration en additif du type sel, lorsque ce dernier est présent, est plus particulièrement comprise entre 0,05 et 1 mol/l, de préférence 0,1 à 0,4 mol/l.

La concentration en additif du type sucre et/ou polysaccharide est telle que la pression osmotique de la phase aqueuse interne comprenant le sucre et/ou polysaccharide correspond à la pression osmotique d'une phase aqueuse interne comprenant 0,05 à 1 mol/l de sel.

La phase organique interne de l'émulsion est choisie parmi les composés non miscibles dans une phase aqueuse. Par non miscible, on entend des composés dont la solubilité dans une phase aqueuse ne dépasse pas 10 % en poids, dans une gamme de température comprise entre 20°C et la température de préparation de l'émulsion inverse et de l'émulsion multiple.

Par ailleurs, les composés utilisés comme phase organique interne sont avantageusement choisis parmi ceux qui se trouvent sous une forme liquide à la température de préparation de l'émulsion inverse et de l'émulsion multiple.

Selon une première possibilité, la phase organique interne comprend au moins une huile organique, d'origine animale ou végétale, ou minérale, ainsi que des cires provenant des mêmes origines, ou leurs mélanges.

Comme huiles organiques d'origine animale, on peut citer en autres, l'huile de cachalot, l'huile de baleine, l'huile de sardine, l'huile de hareng, l'huile de squale, l'huile de foie de morue ; les graisses de porc, de mouton (suifs).

En tant que cires d'origine animale, on peut citer la cire d'abeille.

A titres d'exemples d'huiles organiques d'origine végétale, on peut mentionner, entre autres, l'huile de colza, l'huile de tournesol, l'huile d'arachide, l'huile d'olive, l'huile de noix, l'huile de maïs, l'huile de soja, l'huile de lin, l'huile de chanvre, l'huile de pépins de raisin, l'huile de coprah, l'huile de palme, l'huile de graines de coton, l'huile de babassu, l'huile de jojoba, l'huile de sésame, l'huile de ricin, le beurre de karité, la beurre de cacao.

En tant que cires d'origine végétale, on peut citer la cire de carnauba.

En ce qui concerne les huiles minérales, on peut citer entre autres les huiles naphténiques, paraffiniques (vaseline), les polybutènes (par exemple, obtenues par polymérisation de la coupe C dont la proportion en isobutène est élevée ; gammes Napvis, Hyvis de BP).

Les cires paraffiniques peuvent de même convenir à la préparation de l'émulsion.

Les produits issus de l'alcoolyse des huiles ou cires précitées peuvent aussi être utilisés.

On ne sortirait du cadre de la présente invention en mettant en oeuvre, en tant que phase organique interne, au moins un acide gras, saturé ou non, au moins un ester d'acide gras, saturé ou non, au moins un alcool gras, saturé ou non, ou leurs mélanges.

Plus particulièrement, lesdits acides, esters ou alcools comprennent 8 à 40 atomes de carbone dans la chaîne carbonée la plus longue, plus particulièrement au moins 10 atomes de carbone, de préférence au moins 18 atomes de carbone, et peuvent comprendre une ou plusieurs insaturations éthyléniques, conjuguées ou non. Par ailleurs, les acides, esters ou alcools, peuvent comprendre un ou plusieurs groupements hydroxyle.

Comme exemples d'acides gras saturés, on peut citer les acides palmitique, stéarique, béhénique.

Comme exemples d'acides gras insaturés, on peut citer les acides myristoléique, palmitoléique, oléique, érucique, linoléique, linoléniqué, arachidonique, ricinoléique, ainsi que leurs mélanges.

Comme esters d'acides gras, on peut citer les esters des acides précédemment listés, pour lesquels la partie dérivant de l'alcool comprend 1 à 6 atomes de carbone, comme les esters de méthyle, d'éthyle, de propyle, d'isopropyle, etc.

Comme exemple d'alcools, on peut citer ceux correspondants aux acides précités.

La phase organique interne peut de même être choisie parmi les mono-, di- et triglycérides.

Les huiles essentielles peuvent aussi être utilisées en tant que phase organique interne. Parmi les composés de ce type, on peut citer sans intention de s'y limiter, les huiles et/ou essences de menthe, de menthe verte, de menthe poivrée, de menthol, de vanille, de cannelle, de laurier, d'anis, d'eucalyptus, de thym, de sauge, de feuille de cèdre, de noix de muscade, de citrus (citron, citron vert, pamplemousse, orange), de fruits (pomme, poire, pêche, cerise, prune, fraise, framboise, abricot, ananas, raisin, etc.), seules ou en mélanges.

La phase organique interne peut aussi être choisie parmi les huiles silicones.

Selon un mode de réalisation particulier de l'invention, la phase organique interne comprend au moins une matière active hydrophobe. De préférence, ladite matière active hydrophobe est choisie parmi les espèces compatibles avec la matière active hydrophile présente dans la phase aqueuse interne et, si elle est présente, dans la phase aqueuse externe.

Il est à noter que la phase organique elle-même peut constituer la matière active hydrophobe.

Lesdites matières actives hydrophobes se présentent sous forme liquide, solubilisée dans un solvant organique miscible à la phase organique interne, ou encore sous la forme d'un solide dispersé dans ladite phase.

Plus particulièrement, les matières actives sont telles que leur solubilité dans l'eau ne dépasse pas 10 % en poids, dans une gamme de température comprise entre 20°C et la température de préparation de l'émulsion inverse et de l'émulsion multiple.

Par ailleurs, les matières actives dont le point de fusion est inférieur ou égal à 100°C, plus particulièrement inférieur ou égal à 80°C, peuvent être utilisées.

A titre d'exemple de matières actives utilisables dans le domaine de la cosmétique on peut citer les huiles silicones appartenant par exemple à la famille des diméthicones ; les vitamines lipophiles, comme la vitamine A et ses dérivés notamment ses esters comme l'acétate, le palmitate, le propionate, la vitamine B2, l'acide pantothénique, la vitamine D et la vitamine E ; les mono-, di- et triglycérides ; les bactéricides ; les agents absorbeurs d'UV, comme les dérivés aminobenzoate de type PABA et PARA, les salicylates, les cinnamates, les anthranilates, les dibenzoylméthanes, les dérivés du camphre et leurs mélanges.

Les agents anti-vieillissement peuvent de même être utilisés. A titre d'exemples de tels agents on peut citer notamment citer les rétinoïdes, les vitamines liposolubles, les dérivés de la vitamine C comme les esters notamment l'acétate, le propionate, le palmitate ; les céramides, les pseudo-céramides, les phospholipides, les acides gras, les alcools gras, le cholestérol, les stérols et leurs mélanges. Comme acides gras et alcools préférés, on peut plus particulièrement citer ceux possédant des chaînes alkyles, linéaires ou ramifiées, contenant de 12 à 20 atomes de carbone. Il peut notamment s'agir d'acide linoléique.

On peut de même mettre en oeuvre des agents anti-cellulite, tels que notamment l'isobutylméthylxanthine et la théophyline ; ainsi que des agents anti-acné, comme par exemple le résorcinol, l'acétate de résorcinol, le peroxyde benzoyle et de nombreux composés naturels.

Les arômes, huiles essentielles, parfums peuvent aussi être utilisés en tant que matière active hydrophobe. On pourra se référer aux listes de composés indiquées auparavant.

Les agents anti-microbiens peuvent être choisis parmi le thymol, le menthol, le triclosan, le 4-hexylrésorcinol, le phénol, l'eucalyptol, l'acide benzoïque, le peroxyde benzoïque, le parabène de butyle, et leurs mélanges.

A titre d'exemple de matières actives convenables pour la réalisation de l'invention, dans le domaine des peintures, on peut citer les résines alkydes, les résines époxy, les (poly)isocyanates masqués ou non.

Dans le domaine du papier, on peut citer à titre d'exemples les résines de collage et d'hydrofugation, telles que le dimère d'alkylcétène (AKD) ou l'anhydride alcényle succinique (ASA).

Dans le domaine de l'agrochimie, les matières actives phytosanitaires peuvent être choisies parmi la famille des α-cyano-phénoxybenzyl carboxylates ou des α-cyano-halogénophénoxy-carboxylates, la famille des N-méthylcarbonates comprenant des substituants aromatiques, les matières actives telles que Aldrin, Azinphos-methyl, Benfluralin, Bifenthrin, Chlorphoxim, Chlorpyrifos, Fluchloralin, Fluroxypyr, Dichlorvos, Malathion, Molinate, Parathion, Permethrin, Profenofos, Propiconazole, Prothiofos, Pyrifenox, Butachlor, Metolachlor, Chlorimephos, Diazinon, Fluazifop-P-butyl, Heptopargil, Mecarbam, Propargite, Prosulfocarb, Bromophos-ethyl, Carbophenothion, Cyhalothrin.

Dans le domaine de la détergence, on peut mentionner en tant que matières actives possibles les antimousses silicones.

Il est de même possible d'utiliser des matières actives telles que celles entrant dans la composition de lubrifiants pour le travail ou la déformation des matériaux. La matière active est habituellement une huile, un dérivé d'une huile ou encore un ester d'acide gras.

La matière active peut aussi être choisie parmi les solvants organiques ou les mélanges de tels solvants non miscibles dans l'eau (solubilité dans l'eau ne dépassant pas 10 % en poids, dans une gamme de température comprise entre 20°C et la température de préparation de l'émulsion inverse et de l'émulsion multiple), comme notamment ceux mis en oeuvre pour le nettoyage ou le décapage, tels que les coupes pétrolières aromatiques, les composés terpéniques comme les D- ou L- limonènes, ainsi que les solvants comme le Solvesso®. Conviennent aussi comme solvants, les esters aliphatiques, comme les esters méthyliques d'un mélange d'acides acétique, succinique et glutarique (mélange d'acides sous-produits de la synthèse du Nylon) ; mais aussi les huiles hydrocarbonées telles que l'huile de vaseline, ainsi que les solvants chlorés.

Au cas où la phase organique interne comprend une ou plusieurs matières actives hydrophobes, différentes de la phase organique, leur teneur totale représente plus particulièrement 10 à 50 % en poids de ladite phase organique interne.

L'émulsion inverse comprend en outre au moins un tensioactif et/ou au moins un polymère amphiphile internes.

Selon une première variante, le tensioactif et/ou le polymère amphiphile satisfont à la règle de Bancroft. En d'autres termes, la fraction du tensioactif ou du polymère soluble dans la phase organique interne (phase continue de l'émulsion inverse) est supérieure à la fraction du tensioactif ou du polymère soluble dans la phase aqueuse interne (phase dispersée de l'émulsion inverse). Dans ce cas de figure, on dira que le tensioactif et/ou le polymère se trouve(nt) plutôt situé(s) dans la phase organique interne.

Plus particulièrement, ces tensioactifs et polymères satisfaisant la règle de Bancroft sont choisis parmi les composés de ce type qui vérifient à la fois les deux conditions ci-dessous :
- lorsqu'ils sont mélangés avec la phase organique interne (soit la phase continue de l'émulsion inverse), à une concentration comprise entre 0,1 et 10 % en poids de ladite phase et entre 20 et 30°C, se trouvent sous la forme d'une solution dans tout ou partie de la gamme de concentration indiquée.
- lorsqu'ils sont mélangés avec la phase aqueuse interne (soit la phase dispersée de l'émulsion inverse), à une concentration comprise entre 0,1 et 10 % en poids de ladite phase et entre 20 et 30°C, se trouvent sous la forme d'une dispersion dans tout ou partie de la gamme de concentration indiquée.

Plus particulièrement, le tensioactif et/ou polymère amphiphile sont choisis parmi les tensioactifs non ioniques et/ou les polymères amphiphiles à blocs.

A titre d'exemples de tensioactifs non ioniques susceptibles d'entrer dans la composition de l'émulsion inverse, on peut citer, seuls ou en mélange, les tensioactifs choisis parmi :
- les alcools gras alcoxylés
- les mono, di et triglycérides alcoxylés
- les acides gras alcoxylés
- les esters de sorbitan éventuellement alcoxylés
- les amines grasses alcoxylées
- les di(phényl-1 éthyl) phénols alcoxylés
- les tri(phényl-1 éthyl) phénols alcoxylés
- les alkyls phénols alcoxylés
le nombre de motifs alcoxylés (éthoxylés, propoxylés, butoxylés ou leurs mélanges) est tel que la valeur de HLB soit inférieure ou égale à 8.

Les alcools gras alcoxylés comprennent généralement de 6 à 22 atomes de carbone, les motifs alcoxylés étant exclus de ces nombres.

Les mono, di et triglycérides alcoxylés peuvent être des mono, di et triglycérides d'origine végétale ou animale.

Les esters de sorbitan éventuellement alcoxylés sont des esters du sorbitol cyclisés d'acide gras comprenant de 10 à 20 atomes de carbone comme l'acide laurique, l'acide stéarique ou l'acide oléïque.

Les amines grasses alcoxylées ont généralement de 10 à 22 atomes de carbone, les motifs alcoxylés étant exclus de ces nombres.

Les alkylphénols alcoxylés ont généralement un ou deux groupes alkyles, linéaires ou ramifiés, ayant 4 à 12 atomes de carbone. A titre d'exemples, on peut citer notamment les groupes octyle, nonyle ou dodécyle.

Quant au polymère amphiphile à blocs, celui-ci comprend au moins deux blocs et plus particulièrement au moins un bloc hydrophobe et au moins un bloc hydrophile neutre, anionique ou cationique.

Au cas où le polymère amphiphile comprend au moins trois blocs, et plus particulièrement trois blocs, le polymère est de préférence linéaire. En outre, les blocs hydrophobes se trouvent avantageusement aux extrémités.

Au cas où les polymères comprennent plus de trois blocs, ces derniers sont de préférence sous la forme de polymères greffés ou peignes.

Dans ce qui suit, même si cela constitue un abus de langage, le terme polymère amphiphile à blocs sera utilisé indifféremment pour les polymères linéaires à blocs et les polymères greffés ou peignes.

Lesdits polymères amphiphiles peuvent de manière avantageuse, être obtenus par polymérisation radicalaire dite vivante ou contrôlée. A titre d'exemples non limitatifs de procédés de polymérisation dite vivante ou contrôlée, on peut notamment se référer aux demandes WO 98/58974 (xanthate), WO 97/01478 (dithioesters), WO 99/03894 (nitroxydes) ; WO 99/31144 (dithiocarbamates).

Les polymères amphiphiles peuvent aussi être obtenus par polymérisation cationique, anionique.

Ils peuvent de même être préparés en mettant en jeu des polymérisations par ouverture de cycle (notamment anionique ou cationique), ou par modification chimique du polymère.

Les polymères greffés ou peignes peuvent encore être obtenus par des méthodes dites de greffage direct et copolymérisation.

Le greffage direct consiste à polymériser le(s) monomère(s) choisi(s) par voie radicalaire, en présence du polymère sélectionné pour former le squelette du produit final. Si le couple monomère/squelette ainsi que les conditions opératoires, sont judicieusement choisis, alors il peut y avoir réaction de transfert entre le macroradical en croissance et le squelette. Cette réaction produit un radical sur le squelette et c'est à partir de ce radical que croît le greffon. Le radical primaire issu de l'amorceur peut également contribuer aux réactions de transfert.

Pour ce qui a trait à la copolymérisation, elle met en oeuvre dans un premier temps le greffage à l'extrémité du futur segment pendant, d'une fonction polymérisable par voie radicalaire. Ce greffage peut être réalisé par des méthodes usuelles de chimie organique. Puis, dans un second temps, le macromonomère ainsi obtenu est polymérisé avec le monomère choisi pour former le squelette et on obtient un polymère dit "peigne".

Parmi les monomères hydrophobes à partir desquels le ou les blocs hydrophobes du polymère amphiphile peuvent être préparés, on peut citer notamment :
- les esters des acides mono- ou poly- carboxyliques, linéaires, ramifiés, cycliques ou aromatiques, comprenant au moins une insaturation éthylénique,
- les esters d'acides carboxyliques saturés comprenant 8 à 30 atomes de carbone, éventuellement porteurs d'un groupement hydroxyle ;
- les nitriles αβ-éthyléniquement insaturés, les éthers vinyliques, les esters vinyliques, les monomères vinylaromatiques, les halogénures de vinyle ou de vinylidène,
- les monomères hydrocarbonés, linéaires ou ramifiés, aromatiques ou non, comprenant au moins une insaturation éthylénique,
- les monomères de type siloxane cyclique ou non, les chlorosilanes ;
- l'oxyde de propylène, l'oxyde de butylène ;
seuls ou en mélanges, ainsi que les macromonomères dérivant de tels monomères.

A titre d'exemples particuliers de monomères hydrophobes susceptibles d'entrer dans la préparation du ou des blocs hydrophobes du polymère amphiphile à blocs, on peut citer :
- les esters d'acide (méth)acrylique avec un alcool comprenant 1 à 12 atomes de carbone comme le (méth)acrylate de méthyle, le (méth)acrylate d'éthyle, le (méth)acrylate de propyle, le (méth)acrylate de n-butyle, le (méth)acrylate de t-butyle, le (méth)acrylate d'isobutyle, l'acrylate de 2-éthylhexyl ;
- l'acétate de vinyle, le Versatate® de vinyle, le propionate de vinyle, le chlorure de vinyle, le chlorure de vinylidène, le méthyl vinyléther, l'éthyl vinyléther ;
- les nitriles vinyliques incluent plus particulièrement ceux ayant de 3 à 12 atomes de carbone, comme en particulier l'acrylonitrile et le méthacrylonitrile ;
- le styrène, l'α-méthylstyrène, le vinyltoluène, le butadiène, le chloroprène ;
seuls ou en mélanges, ainsi que les macromonomères dérivant de tels monomères.

Les monomères préférés sont les esters de l'acide acrylique avec les alcools linéaires ou ramifiés en C₁-C₄ tels que l'acrylate de méthyle, d'éthyle, de propyle et de butyle, les esters vinyliques comme l'acétate de vinyle, le styrène, l'α-méthylstyrène.

En ce qui concerne les monomères hydrophiles non ioniques à partir desquels les polymères amphiphiles à blocs peuvent être obtenus, on peut mentionner, sans intention de s'y limiter, l'oxyde d'éthylène ; les amides des acides mono- ou polycarboxyliques, linéaires, ramifiés, cycliques ou aromatiques, comprenant au moins une insaturation éthylénique, ou dérivés, comme le (méth)acrylamide, le N-méthylol (méth)acrylamide ; les esters hydrophiles dérivant de l'acide (méth)acrylique comme par exemple le (méth)acrylate de 2-hydroxyéthyle ; les esters vinyliques permettant d'obtenir des blocs alcool polyvinylique après hydrolyse, comme l'acétate de vinyle, le Versatate® de vinyle, le propionate de vinyle. Ces monomères peuvent être utilisés seuls, en combinaison, ainsi que sous la forme de macromonomères. Il est rappelé que le terme macromonomère désigne une macromolécule portant une ou plusieurs fonctions polymérisables par la méthode mise en oeuvre.

Toutefois les monomères hydrophiles préférés sont l'acrylamide et le méthacrylamide, seuls ou en mélange, ou sous la forme de macromonomères.

En ce qui concerne les monomères hydrophiles anioniques à partir desquels les polymères amphiphiles à blocs peuvent être obtenus, on peut mentionner, par exemple les monomères comprenant au moins une fonction carboxylique, sulfonique, sulfurique, phosphonique, phosphorique, sulfosuccinique, ou les sels correspondants.

Il est précisé que dans les conditions de pH d'utilisation du polymère amphiphile à blocs, les fonctions du ou des blocs anioniques du polymère se trouvent sous une forme au moins partiellement ionisée (dissociée). Plus particulièrement, au moins 10 % en mole des fonctions du ou des blocs sont sous forme ionisée. La détermination de cette valeur ne pose pas de problème à l'homme de l'art ; elle est notamment fonction du pKa des fonctions ionisables des motifs du polymère et du nombre de ces fonctions (soit du nombre de moles de monomères portant des fonctions ionisables mis en oeuvre lors de la préparation du polymère).

Plus particulièrement, les monomères sont choisis parmi :
- les acides mono- ou poly- carboxyliques linéaires, ramifiés, cycliques ou aromatiques, les dérivés N-substitués de tels acides ; les monoesters d'acides polycarboxyliques, comprenant au moins une insaturation éthylénique ;
- les acides vinyl carboxyliques linéaires, ramifiés, cycliques ou aromatiques;
- les aminoacides comprenant une ou plusieurs insaturations éthyléniques ;
seuls ou en mélanges, leurs précurseurs, leurs dérivés sulfoniques ou phosphoniques, ainsi que les macromonomères dérivant de tels monomères ; les monomères ou macromonomères pouvant être sous la forme de sels.

A titre d'exemples de monomères anioniques, on peut citer sans intention de s'y limiter :
- l'acide acrylique, l'acide méthacrylique, l'acide fumarique, l'acide itaconique, l'acide citraconique, l'acide maléique, l'acide acrylamido glycolique, l'acide 2-propène 1-sulfonique, l'acide méthallyl sulfonique, l'acide styrène sulfonique, l'acide α-acrylamido méthylpropane sulfonique, le 2-sulfoéthylène méthacylate, l'acide sulfopropyl acrylique, l'acide bis-sulfopropyl acrylique, l'acide bis-sulfopropyl méthacrylique, l'acide sulfatoéthyl méthacrylique, le monoester phosphate d'acide hydroxyéthyl méthacrylique, ainsi que les sels de métal alcalin, comme le sodium, le potassium, ou d'ammonium ;
- l'acide vinyl sulfonique, l'acide vinylbenzène sulfonique, l'acide vinyl phosphonique, l'acide vinylidène phosphorique, l'acide vinyl benzoïque, ainsi que les sels de métal alcalin, comme le sodium, le potassium, ou d'ammonium ;
- le N-méthacryloyl alanine, le N-acryloyl-hydroxy-glycine ;
seuls ou en mélanges, ainsi que les macromonomères dérivant de tels monomères.

On ne sortirait pas du cadre de la présente invention en mettant en oeuvre des monomères précurseurs de ceux qui viennent d'être cités. En d'autres termes, ces monomères présentent des motifs qui, une fois incorporés dans la chaîne polymère, peuvent être transformés, notamment par un traitement chimique tel que l'hydrolyse, pour redonner les espèces anioniques précitées. Par exemple, les monomères totalement ou partiellement estérifiés des monomères précités peuvent être mis en oeuvre pour être, par la suite, hydrolysés totalement ou en partie.

En tant que monomères hydrophiles cationiques à partir desquels les polymères amphiphiles à blocs peuvent être obtenus, on peut citer notamment :
- les (méth)acrylates d'aminoalkyle, les (méth)acrylamides d'aminoalkyle ;
- les monomères comprenant au moins une fonction amine secondaire, tertiaire ou quaternaire, ou un groupe hétérocyclique contenant un atome d'azote, la vinylamine, l'éthylène imine ;
- les sels d'ammonium de diallyldialkyl;
seuls ou en mélanges, ou les sels correspondants, ainsi que les macromonomères dérivant de tels monomères.

Lesdits monomères peuvent présenter un contre-ion choisi parmi les halogénures comme par exemple le chlore, les sulfates, les hydrosulfates, les alkylsulfates (par exemple comprenant 1 à 6 atomes de carbone), les phosphates, les citrates, les formiates, les acétates.

A titre d'exemples de monomères cationiques convenables figurent entre autres les monomères suivants :
- diméthyl amino éthyl (méth)acrylate, diméthyl amino propyl (méth)acrylate le ditertiobutyl aminoéthyl (méth)acrylate, le diméthyl amino méthyl (méth)acrylamide, le diméthyl amino propyl (méth)acrylamide ;
- l'éthylène imine, la vinylamine, la 2-vinylpyridine, la 4-vinylpyridine ;
- le chlorure de triméthylammonium éthyl (méth)acrylate, le méthyl sulfate de triméthylammonium éthyl acrylate, le chlorure de benzyl diméthylammonium éthyl (méth)acrylate, le chlorure de 4-benzoylbenzyl diméthyl ammonium éthyl acrylate, le chlorure de triméthyl ammonium éthyl (méth)acrylamido, le chlorure de triméthyl ammonium de vinylbenzyle ;
- le chlorure d'ammonium de diallyldiméthyl ;
seuls ou en mélanges, ou leurs sels correspondants, ainsi que les macromonomères dérivant de tels monomères.

De préférence, les polymères amphiphiles à blocs présentent une masse molaire en poids inférieure ou égale à 100000 g/mol, plus particulièrement comprise entre 1000 et 50000 g/mol, de préférence entre 1000 et 20000 g/mol. Il est précisé que les masses molaires en poids indiquées ci-dessus sont des masses molaires théoriques, évaluées en fonction des quantités respectives des monomères introduites lors de la préparation desdits polymères.

De préférence, on met en oeuvre un polymère amphiphile à blocs de type non ionique.

A titre d'exemples de polymère amphiphile à blocs convenant à la mise en oeuvre de l'invention, on peut citer les polymères triblocs polyhydroxystéarate - polyéthylène glycol - polyhydroxystéarate (les produits de la gamme Arlacel de ICI en sont un exemple), les polymères à blocs polydiméthylsiloxane greffé polyéther polyalkyle (comme les produits de la marque Tegopren commercialisés par Goldschmidt).

Selon une deuxième variante, on met en oeuvre, comme tensioactif interne, au moins un tensioactif cationique. Dans le cas de cette deuxième variante, le tensioactif interne ne satisfait pas la règle de Bancroft précédemment énoncée. En effet, le tensioactif cationique est soluble dans la phase dispersée et non dans la phase continue de l'émulsion inverse.

Parmi les tensioactifs cationiques convenables, on peut notamment utiliser les amines grasses aliphatiques ou aromatiques, les amides gras aliphatiques, les dérivés d'ammonium quaternaire (Rhodoquat RP50 de Rhodia Chimie).

Enfin, une troisième variante de l'invention consiste à combiner les deux possibilités qui viennent d'être détaillées.

Quelle que soit la variante retenue, la teneur totale en tensioactif et/ou polymère amphiphile internes représente plus particulièrement de 0,1 à 10 % en poids, de préférence de 2 à 10 % en poids par rapport à la phase aqueuse interne.

Selon un mode de réalisation particulièrement avantageux, l'émulsion inverse comprend un polymère amphiphile ou un mélange de plusieurs d'entre eux.

Par ailleurs, le rapport pondéral phase aqueuse interne / phase organique interne est plus particulièrement compris entre 30/70 et 90/10, de préférence compris entre 50/50 et 90/10.

Pour ce qui a trait à la phase aqueuse externe de l'émulsion multiple, celle-ci peut éventuellement comprendre au moins une matière active hydrophile. Tout ce qui a été mentionné lors de la description des matières hydrophiles susceptibles d'entrer dans la composition de la phase aqueuse interne reste valable dans le cas présent et l'on pourra donc s'y référer.

Si la phase aqueuse externe comprend une ou plusieurs matières actives, leur teneur totale est plus particulièrement comprise entre 0,1 et 50 % en poids de la phase aqueuse externe, et de préférence comprise entre 0,1 et 20 % en poids de la phase aqueuse externe.

La phase aqueuse externe peut de même comprendre des adjuvants supplémentaires comme des agents conservateurs.

La phase aqueuse externe comprend de plus au moins un tensioactif et/ou au moins un polymère amphiphile externes.

Plus particulièrement, les tensioactifs et polymères satisfont la règle de Bancroft et sont de préférence choisis parmi les composés qui vérifient à la fois les deux conditions ci-dessous :
- lorsqu'ils sont mélangés avec la phase aqueuse externe (soit la phase continue de l'émulsion multiple), à une concentration comprise entre 0,1 et 10 % en poids de ladite phase et entre 20 et 30°C, se trouvent sous la forme d'une solution dans tout ou partie de la gamme de concentration indiquée.
- lorsqu'ils sont mélangés avec la phase organique interne (soit la phase dispersée de l'émulsion multiple), à une concentration comprise entre 0,1 et 10 % en poids de ladite phase et entre 20 et 30°C, se trouvent sous la forme d'une dispersion dans tout ou partie de la gamme de concentration indiquée.

Selon une première variante, le tensioactif et/ou polymère amphiphile externes sont choisis parmi les tensioactifs non ioniques, les polymères amphiphiles non ioniques, éventuellement associé(s) à au moins un tensioactif anionique et/ou au moins un polymère amphiphile anionique.

Selon cette première variante, la teneur totale en tensioactif(s) et/ou polymère(s) amphiphile(s) externe(s) est comprise entre 0,5 et 10 % en poids, de préférence entre 1 et 5 % en poids, par rapport à l'émulsion inverse ; la quantité en tensioactif(s) et/ou polymère(s) amphiphile(s) anionique(s), s'ils sont présents, représente 0,5 à 5 % en poids, de préférence 0,5 à 2 % en poids, par rapport au poids de tensioactif(s) et/ou polymère(s) amphiphile(s) non ionique(s).

En ce qui concerne les tensioactifs non ioniques, on met en oeuvre de préférence des tensioactifs non ioniques polyalcoxylés.

De manière avantageuse, le tensioactif non ionique polyalcoxylé est choisi parmi les tensioactifs suivants, seuls ou en mélange :
- les alcools gras alcoxylés
- les mono, di et triglycérides alcoxylés
- les acides gras alcoxylés
- les esters de sorbitan alcoxylés
- les amines grasses alcoxylées
- les di(phényl-1 éthyl) phénols alcoxylés
- les tri(phényl-1 éthyl) phénols alcoxylés
- les alkyls phénols alcoxylés
le nombre de motifs alcoxylés, plus particulièrement éthoxylés et/ou propoxylés, est tel que la valeur de HLB soit supérieure ou égale à 10.

Les tensioactifs cités comme convenant à l'émulsion inverse peuvent être repris, à l'exception du fait que le nombre de motifs éthoxylés et propoxylés, doivent être adaptés en fonction de la valeur de HLB souhaitée. A titre purement illustratif, le nombre total de motifs éthoxylés et éventuellement propoxylés est compris entre 10 et 100.

Quant aux polymères amphiphiles non ioniques convenables et conformément à un premier mode de réalisation, ces derniers sont des composés polyalcoxylés, comprenant au moins deux blocs, l'un d'eux étant hydrophile, l'autre hydrophobe ; l'un au moins des blocs comprenant des motifs polyalcoxylés, plus particulièrement polyéthoxylés et/ou polypropoxylés.

Ce qui a été indiqué auparavant dans le cadre de la description des monomères hydrophiles non ioniques et des monomères hydrophobes utilisables pour la préparation des polymères amphiphiles à blocs entrant dans la composition de l'émulsion inverse, reste valable et ne sera pas repris ici.

A titre purement indicatif, lesdits polymères amphiphiles sont obtenus de préférence en mettant en jeu des polymérisations par ouverture de cycle, notamment anionique.

Plus particulièrement lesdits polymères amphiphiles polyalcoxylés non ioniques sont choisis parmi les polymères dont la masse molaire en poids est inférieure ou égale à 100000 g/mol (mesurée par GPC, étalon polyéthylène glycol), de préférence comprise entre 1000 et 50000 g/mol, de préférence comprise entre 1000 et 20000 g/mol.

A titre d'exemples de polymères de ce type, on peut citer entre autres les polymères triblocs polyéthylène glycol / polypropylène glycol / polyéthylène glycol. De tels polymères sont bien connus et sont notamment commercialisés sous les marques Pluronic (commercialisée par BASF), Arlatone (commercialisée par ICI).

Selon un autre mode de réalisation, le polymère amphiphile non ionique est un polymère amphiphile à blocs, obtenu par polymérisation d'au moins un monomère hydrophile non ionique et d'au moins un monomère hydrophobe, la proportion et la nature desdits monomères étant telles que le polymère résultant vérifie les conditions énoncées auparavant (règle de Bancroft - deux conditions).

Ces polymères amphiphiles comprennent de plus au moins un bloc hydrophobe et au moins un bloc hydrophile neutre (non ionique).

Au cas où ledit polymère comprend au moins trois blocs, et plus particulièrement trois blocs, le polymère est avantageusement linéaire. En outre, les blocs hydrophiles se trouvent plus particulièrement aux extrémités.

Au cas où les polymères comprennent plus de trois blocs, ces derniers sont de préférence sous la forme de polymères greffés ou peignes.

Les listes des monomères hydrophiles non ioniques, des monomères hydrophobes, de même que les diverses méthodes de préparation, citées dans le cadre de la description des polymères amphiphiles à blocs, peuvent être reprises dans le cas des polymères selon cette deuxième variante.

Toutefois les monomères hydrophiles préférés sont l'acrylamide et le méthacrylamide, seuls ou en mélange, ou sous la forme de macromonomères ; les monomères préférés sont les esters de l'acide acrylique avec les alcools linéaires ou ramifiés en C₁-C₄ tels que l'acrylate de méthyle, d'éthyle, de propyle et de butyle, les esters vinyliques comme l'acétate de vinyle, le styrène, l'α-méthylstyrène.

Dans le cas où le tensioactif et/ou polymère amphiphile non ioniques sont associés à au moins un tensioactif et/ou polymère amphiphile anioniques, on peut citer parmi les tensioactifs anioniques convenables, seuls ou en mélanges :
- les alkylesters sulfonates, par exemple de formule R-CH(SO₃M)-COOR', où R représente un radical alkyle en C₈-C₂₀, de préférence en C₁₀-C₁₆, R' un radical alkyle en C₁-C₆, de préférence en C₁-C₃ et M un cation alcalin (sodium, potassium, lithium), ammonium substitué ou non substitué (méthyl-, diméthyl-, triméthyl-, tétraméthylammonium, diméthylpipéridinium ...) ou dérivé d'une alcanolamine (monoéthanolamine, diéthanolamine, triéthanolamine ...). On peut citer tout particulièrement les méthyl ester sulfonates dont le radical R est en C₁₄-C₁₆ ; les alkylbenzènesulfonates, plus particulièrement en C₉-C₂₀, les alkylsulfonates primaires ou secondaires, notamment en C₈-C₂₂, les alkylglycérol sulfonates, les acides polycarboxyliques sulfonés, comme par exemple ceux décrits dans GB 1082179, les sulfonates de paraffine ;
- les alkylsulfates par exemple de formule ROSO₃M, où R représente un radical alkyle ou hydroxyalkyle en C₁₀-C₂₄, de préférence en C₁₂-C₂₀; M représentant un atome d'hydrogène ou un cation de même définition que ci-dessus, ainsi que leurs dérivés polyalcoxylés (éthoxylés (OE), propoxylés (OP), ou leurs combinaisons), comme par exemple le dodécylsulfate de sodium ;
- les alkyléthersulfates par exemple de formule RO(CH₂CH₂O)ₙSO₃M où R représente un radical alkyle ou hydroxyalkyle en C₁₀-C₂₄, de préférence en C₁₂-C₂₀; M représentant un atome d'hydrogène ou un cation de même définition que ci-dessus, n variant généralement de 1 à 4, ainsi que leurs dérivés polyalcoxylés (éthoxylés (OE), propoxylés (OP), ou leurs combinaisons), comme par exemple le laurylethersulfate avec n = 2.
- les alkylamides sulfates, par exemple de formule RCONHR'OSO₃M où R représente un radical alkyle en C₂-C₂₂, de préférence en C₆-C₂₀, R' un radical alkyle en C₂-C₃, M représentant un atome d'hydrogène ou un cation de même définition que ci-dessus, ainsi que leurs dérivés polyalcoxylés (éthoxylés (OE), propoxylés (OP), ou leurs combinaisons) ;
- les sels d'acides gras saturés ou insaturés, par exemple comme ceux en C₈-C₂₄, de préférence en C₁₄-C₂₀, les N-acyl N-alkyltaurates, les alkyliséthionates, les alkylsuccinamates et alkylsulfosuccinates, les monoesters ou diesters de sulfosuccinates, les N-acyl sarcosinates, les polyéthoxycarboxylates ; et
- les phosphates esters d'alkyle et/ou d'alkyléther et/ou d'alkylaryléther.

Parmi les polymères anioniques susceptibles d'être employés, on peut citer tout particulièrement les polymères à blocs, de préférence diblocs ou triblocs, obtenus par polymérisation d'au moins un monomère hydrophile anionique, éventuellement d'au moins un monomère hydrophile non ionique, et d'au moins un monomère hydrophobe.

Là encore, le choix des monomères et leurs proportions respectives sont telles que le polymère résultant vérifie les deux conditions précédemment énoncées (règle de Bancroft).

Les monomères hydrophiles non ioniques, anioniques, les monomères hydrophobes ainsi que les modes de synthèse cités dans le cadre de la description des polymères amphiphiles internes peuvent être mis en oeuvre pour l'obtention des polymères amphiphiles externes selon cette variante. On pourra donc là encore s'y référer.

Une deuxième variante de l'invention consiste à mettre en oeuvre en tant que tensioactif(s) et/ou polymère(s) amphiphile(s) externe(s), au moins un polymère amphiphile anionique éventuellement associé à au moins un tensioactif anionique ; la teneur totale en polymère(s) amphiphile(s) et/ou tensioactif(s) anionique(s) est comprise entre 0,5 et 10 % en poids, de préférence entre 1 et 5 % en poids, par rapport à l'émulsion inverse.

On pourra se reporter aux listes indiquées pour la première variante de tensioactifs et/ou polymères externes pour ce qui concerne les tensioactifs et/ou polymères conformes à la présente variante.

En outre, la phase aqueuse externe peut comprendre au moins un additif dont l'un des rôles est d'équilibrer les pressions osmotiques de la phase aqueuse externe et de la phase aqueuse interne. Parmi les additifs envisageables, on peut citer les sels choisis parmi les halogénures de métaux alcalins ou alcalino-terreux (comme le chlorure de sodium, le chlorure de calcium), au moins un sulfate de métal alcalin ou alcalino-terreux (comme le sulfate de sodium) ou leurs mélanges ; les sucres (glucose par exemple), ou encore les polysaccharides (notamment le dextran) ou leurs mélanges. La phase aqueuse externe peut comprendre une combinaisons de tous ces additifs.

Les concentrations en sel, en sucre et/ou en polysaccharide sont telles que les pressions osmotiques des phases aqueuses externe et interne sont équilibrées.

Selon un mode de réalisation particulier de l'invention, la phase aqueuse externe comprend au moins un composé hydrosoluble ou hydrodispersable, en tant qu'additif de séchage de l'émulsion multiple. En effet, en présence de ces composés, il devient possible de sécher l'émulsion multiple (en d'autres termes d'éliminer l'eau externe de l'adite émulsion) afin d'obtenir des granulés.

Selon une première possibilité, ledit composé est choisi parmi les polymères obtenus par polymérisation d'au moins un monomère (I) acide monocarboxylique ou polycarboxylique, ou anhydride, aliphatique, cyclique ou aromatique, linéaire ou ramifié, éthyléniquement insaturé, et d'au moins un monomère hydrocarboné (II), linéaire ou ramifié, monoéthyléniquement ou polyéthyléniquement insaturé, et/ou d'au moins un monomère (III) ester polyalcoxylé d'acide carboxylique éthyléniquement insaturé.

De préférence, ledit composé est choisi parmi les polymères obtenus par polymérisation d'au moins un monomère (I) et d'au moins un monomère (II). Plus particulièrement, ladite polymérisation est radicalaire.

Selon un mode de réalisation avantageux, le monomère (I) est de formule suivante : (R¹)(R¹) - C = C(R'¹) - COOH ; formule dans laquelle les radicaux R¹, R'¹, identiques ou différents, représentent un atome d'hydrogène, un radical hydrocarboné en C₁ - C₁₀ comprenant éventuellement un groupement - COOH, un groupement - COOH. De préférence, l'un des radicaux R¹ représente un atome d'hydrogène, l'autre radical R¹ représente un atome d'hydrogène, un groupement -COOH ou -(CH₂)-COOH, un radical méthyle, et R'¹, représente un atome d'hydrogène, un groupement -CH₂ COOH ou un radical méthyle.

Le monomère de formule (I) est avantageusement choisi parmi les acides ou anhydrides acrylique, méthacrylique, citraconique, maléique, fumarique, itaconique, crotonique.

Quant au monomère de formule (II), celui-ci correspond plus spécialement à la formule suivante : (R²)(R²) - C = CH₂; dans laquelle les radicaux R², identiques ou différents, représentent un radical hydrocarboné, linéaire ou ramifié, en C₁- C₁₀. Plus particulièrement, lesdits radicaux sont des radicaux alkyles ou alcényle, ces derniers pouvant comprendre une ou plusieurs insaturations éthyléniques. De préférence, lesdits radicaux ne comprennent pas d'hétéroatomes.

Ce monomère peut être notamment choisi parmi l'éthylène, le propylène, le 1-butène, l'isobutylène, le n-1-pentène, le 2-méthyl 1-butène, le n-1-hexène, le 2-méthyl 1-pentène, le 4-méthyl 1-pentène, le 2-éthyl 1-butène, le diisobutylène (ou 2,4,4-triméthyl 1-pentène), le 2-méthyl 3,3-diméthyl 1-pentène.

Le composé hydrosoluble ou hydrodispersable peut de même être choisi parmi les polymères issus de la polymérisation d'au moins un monomère (1) ; parmi les polypeptides d'origine naturelle ou synthétique, ou encore parmi les polysaccharides dépolymérisés obtenus par exemple à partir du dextran, d'amidon, de maltodextrine, de gomme xanthane et de galactomannanes (guar, caroube) et présentant de préférence un point de fusion supérieur à 100°C et une solubilité dans l'eau comprise entre 50 et 500 g/l. Lesdits polymères peuvent éventuellement comprendre au moins un greffon hydrophobe, hydrocarboné, en C₄-C₃₀, saturé ou non aromatique ou non, éventuellement interrompu par un ou plusieurs hétéroatomes.

Il est à noter que les greffons sont reliés au squelette du polymère par l'intermédiaire de liaisons amide, ester, urée, uréthanne, isocyanate, amino, selon la nature des fonctions réactives du polymère à greffer, par exemple des fonctions acide carboxylique, amine, alcool, etc.

De préférence la masse molaire en poids de tels polymères, qu'ils soient ou non greffés, est inférieure ou égale à 20000 g/mol (masses absolues, mesurées par MALLS (Multiangle laser light scattering) couplée à une chromatographie par perméation de gel).

Si un ou plusieurs composés hydrosolubles ou hydrodispersables sont présents, leur teneur totale dans la phase aqueuse externe est de préférence telle que la teneur en ce composé dans l'émulsion multiple séchée (soit dans le granulé final) est comprise entre 30 et 70 % en poids de l'émulsion multiple séchée.

Il est précisé que dans le cas où un tel composé hydrosoluble ou hydrodisoluble est présent, les tensioactifs et/ou polymères externes sont de préférence choisis parmi les tensioactifs et/ou polymères amphiphiles non ioniques de type polyalcoxylés.

Selon une variante de la présente invention, la phase aqueuse externe peut comprendre une phase organique externe dispersée et/ou un solide dispersé.

Tout ce qui a été indiqué précédemment concernant la matière active hydrophobe éventuellement présente dans la phase organique interne, reste valable et ne sera pas détaillé à nouveau maintenant.

Au cas où une telle phase organique externe est présente, elle représente plus particulièrement 1 à 50 % en poids de la phase aqueuse externe, de préférence 5 à 25 % en poids de la phase aqueuse externe.

En outre, il est préférable que la taille des gouttelettes de la phase organique externe soit au plus du même ordre de grandeur que celle de l'émulsion inverse dispersée dans la phase aqueuse externe.

Quant à la possibilité de mettre en oeuvre un solide dispersé dans la phase aqueuse externe, tous les solides utilisés dans les diverses applications mentionnées peuvent convenir. De préférence, la taille de ce solide dispersé est voisine ou plus faible que celle des gouttelettes de l'émulsion inverse.

Au cas où le solide dispersé est présent, sa teneur varie plus particulièrement de 1 à 50 % en poids de la phase aqueuse externe, de préférence de 5 à 25 % en poids.

Selon un mode de réalisation de l'invention, les émulsions multiples sont concentrées. Plus précisément, dans l'émulsion multiple concentrée, le rapport pondéral, émulsion inverse / phase aqueuse externe est compris entre 50/50 et 90/10, de préférence entre 70/30 à 90/10.

Selon une autre possibilité de l'invention, ces émulsions multiples concentrées peuvent être diluées.

L'un des premiers avantages d'une telle étape de dilution est que l'on peut adapter la viscosité des émulsions multiples. En effet, dans certains cas, il est préférable de pouvoir disposer d'émulsions multiples versables.

Un autre avantage à préparer des émulsions multiples plus diluées, et que l'on peut introduire à ce stade de la dilution des additifs comme des agents conservateurs, mais aussi des agents épaississants dont l'effet principal est d'éviter le crémage et/ou la sédimentation de l'émulsion multiple diluée.

Parmi les polymères épaississants convenables, on peut utiliser ceux extraits de végétaux et éventuellement modifiés, tels que les carraghénannes, les alginates, les carboxyméthyl celluloses, les méthylcelluloses, les hydroxypropyl celluloses, les hydroxyéthyl celluloses.

On peut de même employer des polymères épaississants du type des polysaccharides d'origine animale, végétale, bactérienne comme notamment, la gomme xanthane, le guar et dérivés (tels que l'hydroxypropyl guar par exemple), les polydextroses, ou leurs combinaisons.

Lorsqu'il est présent, la teneur en polymère épaississant est plus particulièrement comprise entre 0,1 et 2 % en poids par rapport à la phase aqueuse dans l'émulsion multiple diluée (phase aqueuse externe et phase aqueuse de dilution) de préférence entre 0,1 et 0,5 % en poids par rapport à la phase aqueuse dans l'émulsion multiple diluée. Précisons que dans cette gamme de concentration, le polymère épaississant est soluble dans la phase aqueuse.

Le procédé selon l'invention consiste donc à mettre en oeuvre les étapes suivantes :
a) on prépare l'émulsion inverse ;
b) on prépare la phase aqueuse externe ;
c) on introduit la phase aqueuse externe dans l'émulsion inverse, sans agitation;
d) on agite l'ensemble;
e) on dilue éventuellement l'émulsion multiple concentrée ainsi obtenue par ajout, sous agitation, d'une phase aqueuse de dilution comprenant au moins un tensioactif et/ou au moins un polymère amphiphile externes, afin d'obtenir une émulsion multiple diluée.

L'étape a) du procédé selon l'invention, consistant à préparer l'émulsion inverse, peut être réalisée en mettant en oeuvre des méthodes classiques.

Ainsi, pour ne citer qu'un exemple, on prépare, d'une part, un premier mélange comprenant l'eau, la matière active hydrophile, éventuellement l'additif (sel, sucre, polysaccharide), et éventuellement au moins un tensioactif cationique s'il est présent, et d'autre part, un deuxième mélange comprenant le composé utilisé en tant que phase organique, éventuellement le ou les tensioactifs et/ou le ou les polymères amphiphiles internes, éventuellement la matière active hydrophobe. On ajoute ensuite le premier mélange au second, sous agitation.

Dans le cas où la phase organique est peu visqueuse (viscosité dynamique inférieure à 1 Pa.s, mesurée au moyen d'un viscosimètre Brookfield selon la norme AFNOR NFT 76 102 de février 1972) l'agitation est de préférence forte et peut être, de manière avantageuse, effectuée en utilisant un appareil du type Ultra-Turrax®, Microfluidizer, ou tout homogénéisateur haute pression.

Dans le cas où la phase organique est visqueuse (viscosité dynamique supérieure ou égale à 1 Pa.s ; Brookfield ; norme AFNOR NFT 76 102 de février 1972), l'agitation peut être avantageusement effectuée au moyen d'une pale-cadre, d'un mélangeur de type planétaire, d'un mélangeur possédant un mobile raclant et une pale tournant dans des sens contraires (contre-agitation).

La préparation de l'émulsion inverse est en général réalisée à une température supérieure à la température de fusion du composé utilisé en tant que phase organique. mais inférieure à celle de dégradation des éléments entrant dans la composition de l'emulsion inverse. Plus particulièrement, cette température est comprise entre 20 et 80°C.

La durée de l'agitation peut être déterminée sans difficulté par l'homme de l'art et dépend du type d'appareillage mis en oeuvre. Elle est de préférence suffisante pour obtenir une taille moyenne de gouttelettes de l'émulsion inverse comprise entre 0,1 et 10 µm, de préférence entre 0,1 et 1 µm. La taille moyenne des gouttelettes correspond au diamètre médian en volume (d50) qui représente le diamètre de la particule égal à 50 % de la distribution cumulative, et peut être mesurée soit avec un granulomètre Horiba, soit avec un microscope optique.

L'étape b) du procédé selon l'invention consiste à préparer la phase aqueuse externe. Celle-ci est obtenue par mélange de l'eau, du ou des tensioactifs et/ou polymères amphiphiles externes, éventuellement d'une ou plusieurs matières actives hydrophiles, éventuellement d'au moins un additif (sel, sucre) et le cas échéant, du composé hydrosoluble ou hydrodispersable.

Selon un mode de réalisation avantageux de l'invention, et dans le cas où l'on met en oeuvre une étape de dilution e), l'additif n'est introduit qu'au stade de cette dilution.

Au cas où le composé hydrosoluble ou hydrodispersable est présent, une première variante consisterait à mélanger en premier lieu tous les éléments constitutifs précités, à l'exception dudit composé, pour ne l'introduire qu'une fois le mélange précédent obtenu.

Selon une autre variante, préférée, si le composé hydrosoluble ou hydrodispersable est présent, il n'est introduit qu'au stade de l'étape de dilution e) ou qu'après cette dernière.

Précisions que ladite phase aqueuse externe peut aussi comprendre des adjuvants tels que des agents conservateurs.

La préparation de la phase aqueuse externe peut être effectuée à température ambiante. Cependant, il peut être avantageux de préparer la phase aqueuse externe à une température voisine de celle à laquelle l'émulsion inverse est préparée.

Une fois la phase aqueuse externe obtenue, celle-ci est ajoutée à l'émulsion inverse, lors de l'étape c), sans agitation.

Puis, après avoir introduit la totalité de la phase aqueuse externe dans l'émulsion inverse, on agite l'ensemble.

De manière avantageuse, l'agitation est réalisée au moyen de mélangeurs moyennement cisaillants, comme le sont par exemple les agitateurs munis de pale-cadre, les mélangeurs de type planétaire, ou encore ceux possédant un mobile raclant et une pale tournant dans des sens contraires (contre-agitation).

Cette opération d'agitation a lieu de préférence à une température à laquelle le composé constituant la phase organique interne se trouve sous une forme liquide (température supérieure à la température de fusion du composé constituant la phase organique interne), et plus particulièrement est comprise entre 20 et 80°C.

Il est à noter qu'en fonction de la nature de la ou des matières actives de l'émulsion multiple, ou de certains des éléments constitutifs de ladite émulsion, comme notamment le composé hydrosoluble ou hydrodispersable, il peut être avantageux d'ajuster le pH de la phase aqueuse externe par ajout de base (soude, potasse) ou d'acide (chlorhydrique).

A titre illustratif, la gamme habituelle de pH de la phase aqueuse externe est comprise entre 3 et 8, de préférence entre 5 et 8.

A l'issue de cette étape d'agitation d), on obtient une émulsion multiple concentrée dont le rapport pondéral émulsion inverse / phase aqueuse externe est compris entre 50/50 et 90/10, et de préférence entre 70/30 et 90/10.

Selon les applications, une étape supplémentaire optionnelle de dilution peut être envisagée.

Une telle étape peut être avantageuse, par exemple lorsque l'on souhaite ajouter des composés supplémentaires dans l'émulsion multiple, comme notamment des composés utilisables pour le séchage de l'émulsion multiple, et/ou divers additifs comme des agents épaississants, des agents conservateurs, et/ou une phase organique dispersée dans la phase aqueuse externe, et/ou encore une phase solide dispersée dans cette même phase.

Cette étape de dilution est réalisée en ajoutant, sous agitation, une phase aqueuse de dilution comprenant au moins un tensioactif et/ou au moins un polymère amphiphile externes, qui de préférence sont les mêmes que ceux présents dans la phase externe de l'émulsion multiple concentrée.

De préférence, la quantité de tensioactif et/ou de polymère amphiphile externes dans la phase aqueuse de dilution est telle que la quantité de tensioactif et/ou de polymère amphiphile externes est comprise entre 1 et 10 % en poids par rapport à la phase aqueuse externe de l'émulsion multiple diluée (c'est-à-dire de l'ensemble phase aqueuse de dilution et phase aqueuse externe de l'émulsion multiple concentrée). De préférence cette teneur est comprise entre 1 et 5 % en poids de la phase aqueuse externe de l'émulsion multiple diluée.

Il est à noter que la quantité d'eau de dilution est avantageusement telle que le rapport pondéral émulsion inverse / phase aqueuse externe de l'émulsion multiple diluée est compris entre 10/90 et 50/50. Ainsi, par exemple, une émulsion multiple concentrée présentant un rapport pondéral émulsion inverse / phase aqueuse externe de 90/10 peut être diluée jusqu'à obtenir un rapport pondéral de 50/50 voire de 10/90. Une émulsion multiple concentrée présentant un rapport pondéral émulsion inverse / phase aqueuse externe de 50/50 peut être diluée jusqu'à obtenir un rapport pondéral de 10/90.

En outre, la phase aqueuse de dilution peut comprendre, si nécessaire, au moins un additif (sel, sucre et/ou polysaccharide) dans une quantité telle que la pression osmotique de la phase aqueuse externe de l'émulsion multiple diluée (phase aqueuse externe de l'émulsion multiple concentrée et phase aqueuse de dilution) soit équilibrée avec celle de la phase aqueuse interne.

L'agitation lors de l'étape de dilution est de préférence réalisée au moyen de mélangeurs moyennement cisaillants, tels que ceux mis en oeuvre lors de l'étape d).

Comme mentionné auparavant, dans le cas où l'on souhaite incorporer dans l'émulsion multiple un composé hydrosoluble ou hydrodispersable, cela peut être réalisé soit avec l'eau de dilution ajouter, soit après que la dilution de l'émulsion multiple ait été effectuée, en opérant de telle manière que la teneur en eau ajoutée avec ledit composé hydrosoluble ou hydrodispersable, calculée en fonction que la quantité d'eau ajoutée pour faire l'émulsion multiple diluée, soit telle que le rapport pondéral émulsion inverse / phase aqueuse externe de l'émulsion multiple finale (diluée) entre dans les gammes indiquées auparavant.

Selon une deuxième variante, la phase aqueuse de dilution comprend une phase organique externe dispersée comprenant une matière active hydrophobe.

Dans ce cas, on prépare une émulsion directe comprenant la phase organique externe comprenant la matière active hydrophobe, et la phase aqueuse externe de dilution comprenant le ou les tensioactifs et/ou le ou les polymères amphiphiles externes. Puis on ajoute l'émulsion directe à l'émulsion multiple concentrée précédemment obtenue. Bien évidemment, les quantités de phase aqueuse externe de dilution avec les émulsions inverse et directe sont telles que les proportions pondérales de chacune des phases satisfont aux conditions explicitées ci-dessus pour l'émulsion multiple après dilution. De préférence, les tensioactifs et/ou polymères amphiphiles externes sont les mêmes que ceux de la phase aqueuse externe de l'émulsion multiple concentrée.

L'obtention de l'émulsion directe est réalisée selon toute méthode connue, par mélange sous agitation des deux phases : la phase organique externe comprenant la matière active hydrophobe et la phase aqueuse externe comprenant le tensioactif et/ou polymère amphiphile externes.

Dans le cas où la phase aqueuse externe comprend un solide dispersé, l'obtention de l'émulsion multiple peut être réalisée comme indiqué dans le premier cas, puis on ajoute ledit solide dispersé soit avec la phase aqueuse externe de dilution, soit après l'addition de ladite phase aqueuse externe de dilution.

La taille moyenne des gouttelettes de l'émulsion multiple varie avantageusement entre 5 et 100 µm, plus particulièrement entre 5 et 50 µm, avantageusement entre 10 et 15 µm. La taille moyenne des gouttelettes, correspondant au diamètre médian en volume (d₅₀) qui représente le diamètre de la particule égal à 50 % de la distribution cumulative, est mesurée avec un appareil Horiba et, ou avec un microscope optique.

Dans le cas où un composé hydrosoluble ou hydrodispersable est présent, l'émulsion multiple est ensuite séchée de manière à obtenir des granulés.

L'opération de séchage (consistant à éliminer l'eau de la phase aqueuse externe) peut être effectuée par tout moyen connu de l'homme du métier.

De préférence, le séchage est effectué de telle sorte que qu'au moins 90% en poids de la phase aqueuse externe sont éliminés, de préférence entre 90 et 95 % en poids.

En effectuant le séchage dans de telles conditions, les granulés séchés selon la présente invention comprennent une teneur en eau interne comprise entre 10 et 50 % en poids du granulé, de préférence entre 20 et 30 % en poids du granulé.

Ainsi, selon un premier mode de réalisation de l'invention, on peut envisager un séchage en étuve. De préférence, ce séchage a lieu en couche mince.

Habituellement, la température de séchage est inférieure ou égale à 100°C. Plus particulièrement, des températures comprises entre 50 et 90°C conviennent à la mise en oeuvre de cette méthode.

Selon un autre mode de réalisation, on utilise une méthode de séchage dite rapide, de l'émulsion multiple. Conviennent à ce titre le séchage par atomisation, ou mettant en oeuvre des tambours Duprat®, ou encore la lyophilisation (congélation-sublimation). Le séchage par atomisation peut s'effectuer de manière habituelle dans tout appareil connu tel que par exemple une tour d'atomisation associant une pulvérisation réalisée par une buse ou une turbine avec un courant de gaz chaud.

La température d'entrée des gaz est de l'ordre de 100°C et 120°C et celle de sortie des gaz d'atomisation est de préférence comprise entre 55 et 70°C. Ces températures sont données à titre indicatif, et dépendent de la stabilité thermique des divers éléments. De plus, elle est définie selon la teneur en eau finale souhaitée dans le granulé.

Dans le cas d'opérations de séchage de l'émulsion multiple réalisées au moyen de tambour Duprat®, ou de tout moyen permettant d'obtenir rapidement un film sec qui est séparé du support séchant par une opération de raclage par exemple, on obtient des granulés que l'on peut éventuellement broyer. Si nécessaire, ces granulés peuvent faire l'objet d'une mise en oeuvre ultérieure, comme une étape d'agglomération, de manière à obtenir des granulés agglomérés.

Il est à noter que des additifs, tels que les agents anti-mottants, des charges (comme sillice, kaolin, dioxyde de titane, etc.) peuvent être incorporés aux granulés.

Ces additifs peuvent être introduits avant le séchage de l'émulsion multiple, comme cela peut notamment être le cas des charges. Ils peuvent aussi être co-séchés avec l'émulsion multiple, comme c'est notamment le cas des anti-mottants.

De manière avantageuse, la taille moyenne des granulés (d50) obtenus directement après séchage est comprise entre 100 µm et quelques millimètres (Sympatec), de préférence entre 100 et 800 µm.

Un exemple concret mais non limitatif de l'invention va maintenant être présenté.

### EXEMPLE

### 1/ Emulsion inverse

### Composition de l'émulsion inverse :

* 80% de phase aqueuse interne comprenant le mélange de :
   - 6,25 % d'une solution d'acide lactique (% en poids de solution à 0,1 M exprimé par rapport au poids de phase aqueuse)
   - 93,75 % d'une solution de NaCl (% en poids de solution à 0,1M exprimé par rapport au poids de phase aqueuse)
* 20% de phase huile (huile Primol 352 (*) et agent tensioactif) comprenant 5% d'agent tensioactif (Tegopren 7008 (**); % exprimé en poids par rapport au poids de phase aqueuse interne).
(*) Huile Primol 352 : huile de vaseline blanche médicinale, commercialisée par ESSO SAF
(**) Tegopren 7008: polyméthylsiloxane modifié alkyle et polyéther ; commercialisé par Goldschmidt)

### Préparation de l'émulsion inverse

Dans un réacteur Trimix de 10 litres (Rayneri), muni d'un racleur, contre-agitation et rotor stator, on introduit l'huile Primol et le tensioactif puis on agite l'ensemble, à température ambiante.

On introduit alors, en 80 minutes, la phase aqueuse sous une agitation lente (racleur - 63 tr/mn ; contre-agitation - 190 tr/mn).

Pour affiner l'émulsion, on maintient l'agitation (racleur - 63 tr/mn ; contre-agitation - 190 tr/mn et rotor-stator à 1000 tr/mn), pendant 20 minutes.

On obtient une émulsion inverse visqueuse présentant une granulométrie inférieure au micron (observation faite en microscopie optique).

### 2/ Emulsion multiple concentrée

### Composition de l'émulsion multiple

* 90% d'émulsion inverse
* 10% de phase aqueuse externe contenant :
   - 10% d'Arlatone F127G (*) (ICI - Uniquema ; % en poids exprimé par rapport au poids de phase aqueuse externe de l'émulsion multiple concentrée).
(*) Arlatone F127G : HO(CH₂CH₂O)ₓ(OCH(CH₃)CH₂O)_{y}(CH₂CH₂O)_{z}H.

### Préparation de l'émulsion multiple concentrée

On ajoute à l'émulsion inverse obtenue précédemment, sans agitation et rapidement, la phase aqueuse externe.

On agite ensuite l'ensemble sous agitation (racleur 63 tr/mn et contre-agitation 190 tr/mn)

On obtient une émulsion multiple concentrée dont la taille moyenne des gouttelettes (d50) est voisine de 10 µm avec un indice de polydispersité faible.

### 3/ Emulsion multiple diluée

### Composition de l'émulsion multiple diluée

* 50% d'émulsion inverse
* 50% de phase aqueuse externe contenant :
   - 2% d'Arlatone F127G (% en poids exprimé par rapport au poids de l'émulsion inverse) ;
   - 3,2 % de glucose (% en poids exprimé par rapport au poids de phase aqueuse externe de l'émulsion multiple diluée) ;
   - 1 % de Rhodopol 23 (*) (Rhodia Chimie ; % en poids exprimé par rapport au poids de phase aqueuse externe de l'émulsion multiple diluée).
(*) Rhodopol 23 : gomme xanthane

### Préparation de l'émulsion multiple diluée

### Préparation de la phase aqueuse externe de dilution

La préparation de la phase aqueuse externe se fait en deux temps.

Tout d'abord, on prépare une première solution aqueuse de Rhodopol 23 à 2 %.

On prépare une deuxième solution comprenant de l'eau, l'Arlatone broyé, le glucose et l'agent conservateur (formaldéhyde) en utilisant une agitation de type pale-défloculeuse. Il est précisé que la teneur en eau ajoutée prend en compte la teneur en eau ajoutée pour la préparation de l'émulsion multiple concentrée de manière à obtenir un rapport pondéral émulsion inverse / phase aqueuse totale (phase aqueuse externe et phase aqueuse de dilution) de 50/50.

On mélange ensuite les deux solutions ainsi préparées.

### Préparation de l'émulsion multiple :

On introduit dans l'émulsion multiple concentrée obtenue précédemment, la phase aqueuse externe de dilution préparée, en 10 minutes, à température ambiante et sous agitation lente (racleur 63 tr/mn et contre-agitation 190 tr/mn), et l'on maintient l'agitation 30 minutes après la fin de l'introduction de la phase aqueuse externe de dilution.

On obtient une émulsion multiple diluée présentant la même granulométrie (d50) que celle de l'émulsion multiple concentrée, soit 10 µm.

On a reproduit le même exemple en utilisant une huile polybutène Napvis D107 commercialisée par BP Chemicals et l'on obtient une émulsion multiple diluée de même granulométrie.

## Revendications

1. Procédé de préparation d'une émulsion multiple comprenant une émulsion inverse constituée d'une phase aqueuse interne dispersée dans une phase organique interne, l'émulsion inverse étant dispersée dans une phase aqueuse externe ;
□ la phase aqueuse interne comprenant éventuellement au moins une matière active hydrophile ; la phase organique interne comprenant éventuellement au moins une matière active hydrophobe ;
□ l'émulsion inverse comprenant au moins un tensioactif et/ou au moins un polymère amphiphile interne(s) ;
□ la phase aqueuse externe comprenant éventuellement au moins une matière active hydrophile et au moins un tensioactif et/ou au moins un polymère amphiphile externe(s) ;
**caractérisé en ce que** l'on met en oeuvre les étapes suivantes :
a) on prépare l'émulsion inverse ;
b) on prépare la phase aqueuse externe ;
c) on introduit la phase aqueuse externe dans l'émulsion inverse, sans agitation ;
d) on agite l'ensemble ;
e) on dilue éventuellement l'émulsion multiple concentrée ainsi obtenue, par ajout, sous agitation, d'une phase aqueuse de dilution comprenant au moins un tensioactif et/ou au moins un polymère amphiphile externe, afin d'obtenir une émulsion multiple diluée.

2. Procédé selon la revendication précédente, **caractérisé en ce que** le rapport pondéral phase aqueuse interne / phase organique interne est compris entre 30/70 et 90/10, de préférence compris entre 50/50 et 90/10.

3. Procédé selon l'une des revendications précédentes, **caractérisé en ce que** la phase aqueuse interne comprend au moins un sel choisi parmi les halogénures de métaux alcalins ou alcalino-terreux, ou les sulfates de métal alcalin ou alcalino-terreux, ou au moins un sucre, ou au moins un polysaccharide, ou leurs mélanges.

4. Procédé selon la revendication 3, **caractérisé en ce que** la concentration en sel dans la phase aqueuse interne est comprise entre 0,05 et 1 mol/l, de préférence 0,1 à 0,4 mol/l ; la concentration en sucre et/ou polysaccharide est telle que la pression osmotique de la phase aqueuse interne comprenant le sucre et/ou le polysaccharide correspond à la pression osmotique d'une phase aqueuse interne comprenant 0,05 à 1 mol/l de sel.

5. Procédé selon l'une des revendications précédentes, **caractérisé en ce** le ou les tensioactifs et/ou le ou les polymères amphiphiles internes sont choisis parmi les tensioactifs non ioniques et/ou les polymères amphiphiles à blocs et/ou les tensioactifs cationiques, la teneur totale en tensioactif(s) et/ou polymère(s) amphiphile(s) interne(s) représentant de 0,1 à 10 %, de préférence de 2 à 10 %, en poids de la phase organique interne.

6. Procédé selon l'une des revendications précédentes, **caractérisé en ce que** le rapport pondéral dans l'émulsion multiple concentrée, émulsion inverse / phase aqueuse externe est compris entre 50/50 et 90/10, de préférence entre 70/30 à 90/10.

7. Procédé selon l'une des revendications précédentes, **caractérisé en ce que** la phase aqueuse externe comprend, en tant que tensioactif et/ou polymère amphiphile externes :
- au moins un tensioactif non ionique et/ou au moins un polymère amphiphile non ionique éventuellement associé(s) à au moins un tensioactif anionique et/ou au moins un polymère amphiphile anionique ; la teneur totale en tensioactif(s) et/ou polymère(s) amphiphile(s) externe(s) est comprise entre 0,5 et 10 % en poids, de préférence entre 1 et 5 % en poids, par rapport à l'émulsion inverse ; la quantité en tensioactif(s) et/ou polymère amphiphile(s) anionique(s), s'ils sont présents, représente 0,5 à 5 % en poids, de préférence 0,5 à 2 % en poids, par rapport au poids de tensioactif(s) et/ou polymère(s) amphiphile(s) non ionique(s) ; ou
- au moins un polymère amphiphile anionique éventuellement associé à au moins un tensioactif anionique ; la teneur totale en polymère(s) amphiphile(s) et/ou tensioactif(s) anionique(s) est comprise entre 0,5 et 10 % en poids, de préférence entre 1 et 5 % en poids, par rapport à l'émulsion inverse.

8. Procédé selon l'une des revendications précédentes, **caractérisé en ce que** la quantité de tensioactif et/ou de polymère amphiphile externe(s) dans la phase aqueuse de dilution est telle que la quantité de tensioactif et/ou de polymère amphiphile externe(s) est comprise entre 1 et 10 % en poids par rapport à la phase aqueuse externe de l'émulsion multiple diluée, de préférence entre 1 et 5 % en poids de la phase aqueuse externe de l'émulsion multiple diluée

9. Procédé selon l'une des revendications précédentes, **caractérisé en ce que** la quantité d'eau de dilution est telle que le rapport pondéral émulsion inverse / phase aqueuse externe de l'émulsion multiple diluée est compris entre 10/90 et 50/50.

10. Procédé selon l'une des revendications précédentes, **caractérisé en ce que** la phase aqueuse de dilution comprend au moins un sel choisi parmi les halogénures de métaux alcalins ou alcalino-terreux, d'au moins un sulfate de métal alcalin ou alcalino-terreux, d'au moins un sucre, ou encore d'au moins un polysaccharide, ou leurs mélanges, en quantité telle que la pression osmotique de l'ensemble phase aqueuse externe et phase aqueuse de dilution soit équilibrée avec celle de la phase aqueuse interne.

11. Procédé selon l'une des revendications précédentes, **caractérisé en ce que** la phase aqueuse externe ou la phase aqueuse de dilution comprend au moins un composé hydrosoluble ou hydrodispersable, en tant qu'additif de séchage de l'émulsion multiple, choisi parmi :
* les polymères obtenus par polymérisation d'au moins un monomère (I) acide monocarboxylique ou polycarboxylique, ou anhydride, aliphatique, cyclique ou aromatique, linéaire ou ramifié, éthyléniquement insaturé, et d'au moins un monomère hydrocarboné (II), linéaire ou ramifié, monoéthyléniquement ou polyéthyléniquement insaturé, et/ou d'au moins un monomère (III) ester polyalcoxylé d'acide carboxylique éthyléniquement insaturé ;
* les polymères issus de la polymérisation d'au moins un monomère (I), les polypeptides d'origine naturelle ou synthétique, ou encore les polysaccharides fortement dépolymérisés; lesdits polymères pouvant éventuellement comprendre au moins un greffon hydrophobe, hydrocarboné, en C₄-C₃₀, saturé ou non aromatique ou non, éventuellement interrompu par un ou plusieurs hétéroatomes ;
* la teneur en composé hydrosoluble ou hydrodispersable dans la phase aqueuse externe est telle que la teneur en ce composé dans l'émulsion multiple séchée est comprise entre 30 et 70 % en poids de l'émulsion multiple séchée.

12. Procédé selon l'une des revendications précédentes, **caractérisé en ce que** la phase aqueuse de dilution comprend un agent épaississant choisi parmi les polymères extraits de végétaux et éventuellement modifiés, parmi les polymères du type des polysaccharides d'origine animale, végétale, bactérienne, ou leurs combinaisons ; la teneur en polymère épaississant étant comprise entre 0,1 et 2 % en poids par rapport à la phase aqueuse dans l'émulsion multiple diluée, de préférence entre 0,1 et 0,5 % en poids par rapport à la phase aqueuse dans l'émulsion multiple diluée.

13. Procédé selon l'une des revendications précédentes, **caractérisé en ce que** la phase aqueuse de dilution comprend une phase organique externe dispersée comprenant une matière active hydrophobe.

14. Procédé selon la revendication précédente, **caractérisé en ce que** la phase organique externe dispersée représente 1 à 50 % en poids de la phase externe aqueuse de l'émulsion multiple diluée, de préférence 5 à 25 % en poids de la aqueuse externe de l'émulsion multiple diluée.

15. Procédé selon l'une des revendications précédentes, **caractérisé en ce que** la phase aqueuse de dilution comprend au moins un solide dispersé.

16. Procédé selon la revendication précédente, **caractérisé en ce que** le solide dispersé représente 1 à 50 % en poids de la phase aqueuse externe de l'émulsion multiple diluée, de préférence 5 à 25 % en poids de la phase aqueuse externe de l'émulsion multiple diluée.

## Claims

1. A process for preparing a multiple emulsion comprising an inverse emulsion consisting of an inner aqueous phase dispersed in an inner organic phase, the inverse emulsion being dispersed in an outer aqueous phase;
• the inner aqueous phase optionally comprising at least one hydrophilic active material; the inner organic phase optionally comprising at least one hydrophobic active material;
• the inverse emulsion comprising at least one inner surfactant and/or at least one inner amphiphilic polymer;
• the outer aqueous phase optionally comprising at least one hydrophilic active material and at least one outer surfactant and/or at least one outer amphiphilic polymer;
**characterized in that** the following steps are performed:
a) the inverse emulsion is prepared;
b) the outer aqueous phase is prepared;
c) the outer aqueous phase is introduced into the inverse emulsion, without stirring;
d) the mixture is stirred;
e) the concentrated multiple emulsion thus obtained is optionally diluted, by adding, with stirring, an aqueous dilution phase comprising at least one outer surfactant and/or at least one outer amphiphilic polymer, in order to obtain a dilute multiple emulsion.

2. The process as claimed in the preceding claim, **characterized in that** the inner aqueous phase/inner organic phase weight ratio is between 30/70 and 90/10 and preferably between 50/50 and 90/10.

3. The process as claimed in either of the preceding claims, **characterized in that** the inner aqueous phase comprises at least one salt chosen from alkali metal or alkaline-earth metal halides, or alkali metal or alkaline-earth metal sulfates, or at least one sugar, or at least one polysaccharide, or mixtures thereof.

4. The process as claimed in one of the preceding claims, **characterized in that** the salt concentration in the inner aqueous phase is between 0.05 and 1 mol/l and preferably 0.1 to 0.4 mol/l; the sugar and/or polysaccharide concentration is such that the osmotic pressure of the inner aqueous phase comprising the sugar and/or the polysaccharide corresponds to the osmotic pressure of an inner aqueous phase comprising 0.05 to 1 mol/l of salt.

5. The process as claimed in one of the preceding claims, **characterized in that** the inner surfactant(s) and/or the inner amphiphilic polymer(s) are chosen from nonionic surfactants and/or amphiphilic block polymers and/or cationic surfactants, the total content of inner surfactant(s) and/or inner amphiphilic polymer(s) representing from 0.1% to 10% and preferably from 2% to 10% by weight of the inner organic phase.

6. The process as claimed in one of the preceding claims, **characterized in that** the inverse emulsion/outer aqueous phase weight ratio in the concentrated multiple emulsion is between 50/50 and 90/10 and preferably between 70/30 and 90/10.

7. The process as claimed in one of the preceding claims, **characterized in that** the outer aqueous phase comprises, as outer surfactant and/or outer amphiphilic polymer:
- at least one nonionic surfactant and/or at least one nonionic amphiphilic polymer, optionally combined with at least one anionic surfactant and/or at least one anionic amphiphilic polymer; the total content of outer surfactant(s) and/or outer amphiphilic polymer(s) is between 0.5% and 10% by weight and preferably between 1% and 5% by weight relative to the inverse emulsion; the amount of anionic surfactant(s) and/or anionic amphiphilic polymer(s), if they are present, represents 0.5% to 5% by weight and preferably 0.5% to 2% by weight relative to the weight of nonionic surfactant(s) and/or nonionic amphiphilic polymer (s) ; or
- at least one anionic amphiphilic polymer optionally combined with at least one anionic surfactant; the total content of anionic amphiphilic polymer(s) and/or anionic surfactant(s) is between 0.5% and 10% by weight and preferably between 1% and 5% by weight relative to the inverse emulsion.

8. The process as claimed in one of the preceding claims, **characterized in that** the amount of outer surfactant and/or of outer amphiphilic polymer in the aqueous dilution phase is such that the amount of outer surfactant and/or of outer amphiphilic polymer is between 1% and 10% by weight relative to the outer aqueous phase of the dilute multiple emulsion, and preferably between 1% and 5% by weight of the outer aqueous phase of the dilute multiple emulsion.

9. The process as claimed in one of the preceding claims, **characterized in that** the amount of dilution water is such that the inverse emulsion/outer aqueous phase weight ratio of the dilute multiple emulsion is between 10/90 and 50/50.

10. The process as claimed in one of the preceding claims, **characterized in that** the aqueous dilution phase comprises at least one salt chosen from alkali metal or alkaline-earth metal halides, at least one alkali metal or alkaline-earth metal sulfate, at least one sugar or at least one polysaccharide, or mixtures thereof, in an amount such that the osmotic pressure of the combination of outer aqueous phase and aqueous dilution phase is equilibrated with that of the inner aqueous phase.

11. The process as claimed in one of the preceding claims, **characterized in that** the outer aqueous phase or the aqueous dilution phase comprises at least one water-soluble or water-dispersible compound, as additive for drying the multiple emulsion, chosen from:
* the polymers obtained by polymerization of at least one aliphatic, cyclic or aromatic, linear or branched, ethylenically unsaturated monocarboxylic or polycarboxylic acid or anhydride monomer (I) and of at least one linear or branched, monoethylenically or polyethylenically unsaturated hydrocarbon-based monomer (II), and/or of at least one ethylenically unsaturated polyalkoxylated carboxylic acid ester monomer (III);
* the polymers derived from the polymerization of at least one monomer (I), polypeptides of natural or synthetic origin, or highly depolymerized polysaccharides; said polymers optionally comprising at least one saturated or unsaturated, aromatic or nonaromatic C₄-C₃₀ hydrocarbon-based hydrophobic graft, optionally interrupted with one or more hetero atoms;
* the content of water-soluble or water-dispersible compound in the outer aqueous phase is such that the content of this compound in the dried multiple.emulsion is between 30% and 70% by weight of the dried multiple emulsion.

12. The process as claimed in one of the preceding claims, **characterized in that** the aqueous dilution phase comprises a thickener chosen from polymers extracted from plants and optionally modified, from polymers of the type such as polysaccharides of animal, plant or bacterial origin, or combinations thereof; the content of thickening polymer being between 0.1% and 2% by weight relative to the aqueous phase in the dilute multiple emulsion, and preferably between 0.1% and 0.5% by weight relative to the aqueous phase in the dilute multiple emulsion.

13. The process as claimed in one of the preceding claims, **characterized in that** the aqueous dilution phase comprises a dispersed outer organic phase comprising a hydrophobic active material.

14. The process as claimed in the preceding claim, **characterized in that** the dispersed outer organic phase represents 1% to 50% by weight of the outer aqueous phase of the dilute multiple emulsion and preferably 5% to 25% by weight of the outer aqueous of the dilute multiple emulsion.

15. The process as claimed in one of the preceding claims, **characterized in that** the aqueous dilution phase comprises at least one dispersed solid.

16. The process as claimed in the preceding claim, **characterized in that** the dispersed solid represents 1% to 50% by weight of the outer aqueous phase of the dilute multiple emulsion and preferably 5% to 25% by weight of the outer aqueous phase of the dilute multiple emulsion.

## Patentansprüche

1. Verfahren zur Herstellung einer Mehrfachemulsion, die eine inverse Emulsion umfasst, welche aus einer inneren wässrigen Phase besteht, die in einer inneren organischen Phase dispergiert ist, wobei die inverse Emulsion in einer äußeren wässrigen Phase dispergiert ist; wobei
■ die innere wässrige Phase gegebenenfalls mindestens einen hydrophilen aktiven Stoff umfasst; die innere organische Phase gegebenenfalls mindestens einen hydrophoben aktiven Stoff umfasst;
■ die inverse Emulsion mindestens ein inneres Tensid und/oder mindestens ein inneres amphiphiles Polymer umfasst;
■ die äußere wässrige Phase gegebenenfalls mindestens einen hydrophilen aktiven Stoff und mindestens ein äußeres Tensid und/oder mindestens ein äußeres amphiphiles Polymer umfasst;
**dadurch gekennzeichnet, dass** die folgenden Schritte durchgeführt werden:
a) Herstellung der inversen Emulsion;
b) Herstellung der äußeren wässrigen Phase;
c) Einbringen der äußeren wässrigen Phase in die inverse Phase ohne zu rühren;
d) Rühren des Ganzen;
e) gegebenenfalls Verdünnung der so erhaltenen konzentrierten Mehrfachemulsion durch Zugabe unter Rühren einer wässrigen Phase zur Verdünnung, welche mindestens ein Tensid und/oder mindestens ein äußeres amphiphiles Polymer umfasst, um eine verdünnte Mehrfachemulsion zu erhalten.

2. Verfahren gemäß dem vorhergehenden Anspruch, **dadurch gekennzeichnet, dass** das Gewichtsverhältnis innere wässrige Phase / innere organische Phase zwischen 30/70 und 90/10, vorzugsweise zwischen 50/50 und 90/10 liegt.

3. Verfahren gemäß einem der vorhergehenden Ansprüche, **dadurch gekennzeichnet, dass** die innere wässrige Phase mindestens ein Salz umfasst, das ausgewählt ist aus Alkali- oder Erdalkalimetallhalogeniden oder Alkali- oder Erdalkalimetallsulfaten oder mindestens einem Zucker oder mindestens einem Polysaccharid oder deren Gemischen.

4. Verfahren gemäß Anspruch 3, **dadurch gekennzeichnet, dass** die Salzkonzentration in der inneren wässrigen Phase zwischen 0,05 und 1 mol/l, vorzugsweise zwischen 0,1 und 0,4 mol/l liegt; die Konzentration an Zucker und/oder Polysaccharid derart ist, dass der osmotische Druck der inneren wässrigen Phase, die den Zucker und/oder das Polysaccharid umfasst, dem osmotischen Druck einer inneren wässrigen Phase, die 0,05 bis 1 mol/l Salz umfasst, entspricht.

5. Verfahren gemäß einem der vorhergehenden Ansprüche, **dadurch gekennzeichnet, dass** das oder die inneren Tenside und/oder das oder die inneren amphiphilen Polymere ausgewählt sind aus nicht-ionischen Tensiden und/oder amphiphilen Blockpolymeren und/oder kationischen Tensiden, wobei der Gesamtgehalt an inneren Tensiden und/oder inneren amphiphilen Polymeren 0,1 bis 10 Gew.-%, vorzugsweise 2 bis 10 Gew.-% der inneren organischen Phase darstellt.

6. Verfahren gemäß einem der vorhergehenden Ansprüche, **dadurch gekennzeichnet, dass** in der konzentrierten Mehrfachemulsion das Gewichtsverhältnis inverse Emulsion / äußere wässrige Phase zwischen 50/50 und 90/10, vorzugsweise zwischen 70/30 und 90/10 liegt.

7. Verfahren gemäß einem der vorhergehenden Ansprüche, **dadurch gekennzeichnet, dass** die äußere wässrige Phase als äußeres Tensid und/oder äußeres amphiphiles Polymer umfasst:
- mindestens ein nicht-ionisches Tensid und/oder mindestens ein nicht-ionisches amphiphiles Polymer, das gegebenenfalls mit mindestens einem anionischen Tensid und/oder mindestens einem anionischen amphiphilen Polymer assoziiert ist; wobei der Gesamtgehalt an äußeren Tensiden und/oder äußeren amphiphilen Polymeren zwischen 0,5 und 10 Gew.-%, vorzugsweise zwischen 1 und 5 Gew.-% im Verhältnis zur inversen Emulsion liegt; wobei die Menge an anionischen Tensiden und/oder anionischen amphiphilen Polymeren, falls vorhanden, bei 0,5 bis 5 Gew.-%, vorzugsweise bei 0,5 bis 2 Gew.-% im Verhältnis zum Gewicht des/der nicht-ionischen Tensids/Tenside und/oder nicht-ionischen amphiphilen Polymers/Polymere liegt; oder
- mindestens ein anionisches amphiphiles Polymer, das gegebenenfalls mit mindestens einem anionischen Tensid assoziiert ist; wobei der Gesamtgehalt an amphiphilen Polymeren und/oder anionischen Tensiden zwischen 0,5 und 10 Gew.-%, vorzugsweise zwischen 1 und 5 Gew.-% im Verhältnis zur inversen Emulsion liegt.

8. Verfahren gemäß einem der vorhergehenden Ansprüche, **dadurch gekennzeichnet, dass** die Menge an äußerem Tensid und/oder äußerem amphiphilem Polymer in der wässrigen Phase zur Verdünnung derart ist, dass die Menge an äußerem Tensid und/oder äußerem amphiphilem Polymer zwischen 1 und 10 Gew.-% im Verhältnis zur äußeren wässrigen Phase der verdünnten Mehrfachemulsion, vorzugsweise zwischen 1 und 5 Gew.-% der äußeren wässrigen Phase der verdünnten Mehrfachemulsion liegt.

9. Verfahren gemäß einem der vorhergehenden Ansprüche, **dadurch gekennzeichnet, dass** die Menge an Wasser zur Verdünnung derart ist, dass das Gewichtsverhältnis inverse Emulsion / äußere wässrige Phase der verdünnten Mehrfachemulsion zwischen 10/90 und 50/50 liegt.

10. Verfahren gemäß einem der vorhergehenden Ansprüche, **dadurch gekennzeichnet, dass** die wässrige Phase zur Verdünnung mindestens ein Salz umfasst, das ausgewählt ist aus Alkali- oder Erdalkalimetallhalogeniden, mindestens einem Alkali- oder Erdalkalimetallsulfat, mindestens einem Zucker oder auch mindestens einem Polysaccharid oder deren Gemischen, in einer Menge, so dass der osmotische Druck der Gesamtheit aus äußerer wässriger Phase und wässriger Phase zur Verdünnung im Gleichgewicht mit dem der inneren wässrigen Phase steht.

11. Verfahren gemäß einem der vorhergehenden Ansprüche, **dadurch gekennzeichnet, dass** die äußere wässrige Phase oder die wässrige Phase zur Verdünnung mindestens eine wasserlösliche oder in Wasser dispergierbare Verbindung als Trocknungszusatz der Mehrfachemulsion umfasst, die ausgewählt ist aus:
■ Polymeren, die durch Polymerisation von mindestens einem aliphatischen, zyklischen oder aromatischen, linearen oder verzweigten, ethylenisch ungesättigten Monocarbonsäure- oder Polycarbonsäure- oder Anhydrid-Monomer (I) und mindestens einem linearen oder verzweigten, monoethylenisch oder polyethylenisch ungesättigten Kohlenwasserstoffmonomer (II) und/oder mindestens einem polyalkoxylierten ethylenisch ungesättigten Carbonsäureester-Monomer (III) erhalten wurden;
■ Polymeren, die aus der Polymerisation von mindestens einem Monomer (I), Polypeptiden natürlichen oder synthetischen Ursprungs oder auch stark depolymerisierten Polysacchariden entstanden sind; wobei die besagten Polymere gegebenenfalls mindestens eine hydrophobe, gesättigte oder nicht gesättigte, aromatische oder nicht aromatische C₄-C₃₀-Kohlenwasserstoff-Pfropfgruppe umfassen können, die gegebenenfalls mit einem oder mehreren Heteroatom(en) unterbrochen ist;
■ der Gehalt an wasserlöslicher oder in Wasser dispergierbarer Verbindung in der äußeren wässrigen Phase derart ist, dass der Gehalt dieser Verbindung in der getrockneten Mehrfachemulsion zwischen 30 und 70 Gew.-% der getrockneten Mehrfachemulsion liegt.

12. Verfahren gemäß einem der vorhergehenden Ansprüche, **dadurch gekennzeichnet, dass** die wässrige Phase zur Verdünnung ein Verdickungsmittel umfasst, das ausgewählt ist aus Polymeren, die aus Pflanzen extrahiert und gegebenenfalls modifiziert sind, aus Polymeren vom Typ Polysaccharid tierischen, pflanzlichen, bakteriellen Ursprungs oder deren Kombinationen; wobei der Gehalt an Verdickungspolymer zwischen 0,1 und 2 Gew.-% im Verhältnis zur wässrigen Phase in der verdünnten Mehrfachemulsion, vorzugsweise zwischen 0,1 und 0,5 Gew.-% im Verhältnis zur wässrigen Phase in der verdünnten Mehrfachemulsion liegt.

13. Verfahren gemäß einem der vorhergehenden Ansprüche, **dadurch gekennzeichnet, dass** die wässrige Phase zur Verdünnung eine dispergierte äußere organische Phase umfasst, die einen hydrophoben aktiven Stoff umfasst.

14. Verfahren gemäß dem vorhergehenden Anspruch, **dadurch gekennzeichnet, dass** die dispergierte äußere organische Phase 1 bis 50 Gew.-% der äußeren wässrigen Phase der verdünnten Mehrfachemulsion, vorzugsweise 5 bis 25 Gew.-% der äußeren wässrigen Phase der verdünnten Mehrfachemulsion darstellt.

15. Verfahren gemäß einem der vorhergehenden Ansprüche, **dadurch gekennzeichnet, dass** die wässrige Phase zur Verdünnung mindestens einen dispergierten Feststoff umfasst.

16. Verfahren gemäß dem vorhergehenden Anspruch, **dadurch gekennzeichnet, dass** der dispergierte Feststoff 1 bis 50 Gew.-% der äußeren wässrigen Phase der verdünnten Mehrfachemulsion, vorzugsweise 5 bis 25 Gew.-% der äußeren wässrigen Phase der verdünnten Mehrfachemulsion darstellt.
